# EUROPÄISCHE PATENTANMELDUNG

(11) **EP 1 806 127 A1**
(43) Veröffentlichungstag der Anmeldung: **11.07.2007**
(21) Anmeldenummer: 06025997.5
(22) Anmeldetag: 15.12.2006
(51) Int. Cl.: A61K 8/898, A61K 8/22, A61Q 5/08

(54) **Bleichmittel, enthaltend Polyammonium-Polysiloxan-Verbindung**

(30) Priorität: 23.12.2005 DE 102005062647
(71) Anmelder: Henkel KGaA, 40589 Düsseldorf (DE)
(72) Erfinder: Seiler, Martina, 47228 Duisburg (DE); Reichert, Anja, 40629 Düsseldorf (DE); Goutsis, Konstantin, 41812 Erkelenz (DE)

(57) **Zusammenfassung**

Gegenstand der vorliegenden Anmeldung sind Mittel zur aufhellenden Farbveränderung keratinischer Fasern, insbesondere menschlicher Haare, die in einem kosmetisch akzeptablen Träger mindestens ein Oxidationsmittel, ausgewählt aus Wasserstoffperoxid und dessen Anlagerungsverbindungen an feste Träger, und mindestens einen Bleichkraftverstärker sowie zusätzlich mindestens eine Polyammonium-Polysiloxan Verbindung enthalten.

## Beschreibung

Gegenstand der vorliegenden Anmeldung sind Mittel zur aufhellenden Farbveränderung keratinischer Fasern, die eine Polyammonium-Polysiloxan-Verbindung enthalten, sowie ein Verfahren zur aufhellenden Farbveränderung unter Einsatz des erfindungsgemäßen Mittels.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Dabei können Dauerwell- und andere die Haarform verändernde Verfahren nahezu unabhängig vom Typ der zu behandelnden Haare eingesetzt werden. Dagegen sind Färbe- und insbesondere Blondierverfahren auf bestimmte Ausgangshaarfarben begrenzt. So eignen sich für aufhellende Verfahren, die so genannten Blondierverfahren, im Wesentlichen nur dunkelblonde oder hellere Haare. Die Grundlagen der Blondierverfahren sind dem Fachmann bekannt und in einschlägigen Monographien, z.B. von K. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, zusammenfassend beschrieben.

Zum Blondieren menschlicher Haare - insbesondere für die Strähnchenapplikation -werden üblicherweise feste oder pastenförmige Zubereitungen mit festen Oxidationsmitteln unmittelbar vor der Anwendung mit einer verdünnten Wasserstoffperoxidlösung vermischt. Diese Mischung wird dann auf das Haar aufgebracht und nach einer bestimmten Einwirkzeit wieder ausgespült.

Parallel zu dem Bedürfnis, die Haarfarbe zu verändern, besteht üblicherweise der Wunsch, den Pflegezustand der Fasern nicht zu vernachlässigen. Daher werden herkömmlicher Weise die Fasern unmittelbar nach der farbverändernden Behandlung mit einem separat formulierten Pflegemittel behandelt.

In jüngster Zeit gab es stets Bestrebungen, die pflegenden Komponenten bereits mit in die aufhellende Zubereitung einzuarbeiten. Derartige Versuche scheiterten in der Vergangenheit häufig daran, dass die eingesetzten Pflegestoffe unter den aggressiven Bedingungen der aufhellenden Zubereitung nicht ausreichend stabil sind.

Ferner traten häufig Probleme bei der Einarbeitung der Pflegestoffe in die Anwendungszubereitungen auf, wie beispielsweise Entmischungsphänomene.

Aufgabe der vorliegenden Erfindung war es somit ein aufhellendes farbveränderndes Mittel für keratinische Fasern zur Verfügung zu stellen, das stabil formuliert werden kann und gleichzeitig eine intensive Pflege der Fasern unmittelbar im farbverändernden Schritt ermöglicht.

Ein erster Gegenstand der vorliegenden Erfindung sind daher Mittel zur aufhellenden Farbveränderung keratinischer Fasern, insbesondere menschlicher Haare, enthaltend in einem kosmetisch akzeptablen Träger mindestens ein Oxidationsmittel, ausgewählt aus Wasserstoffperoxid und dessen Anlagerungsverbindungen an feste Träger, und mindestens einen Bleichkraftverstärker, die zusätzlich mindestens eine Polyammonium-Polysiloxan Verbindung enthalten.

Unter keratinhaltigen Fasern werden erfindungsgemäß Pelze, Wolle, Federn und insbesondere menschliche Haare verstanden.

Die erfindungsgemäßen Mittel enthalten mindestens eine Polyammonium-Polysiloxan Verbindung, die wie im Folgenden beschrieben, aufgebaut ist. Die Polyammonium-Polysiloxan Verbindungen enthalten:

### a1) mindestens eine Polyalkylenoxid-Struktureinheit der allgemeinen Formeln: -A-E-, -E-A-, -A-E-A'- und/oder -A'-E-A- , worin:

A steht für eine der Gruppen: -CH₂C(O)O-, -CH₂CH₂C(O)O-, -CH₂CH₂CH₂C(O)O-, -OC(O)CH₂-, -OC(O)CH₂CH₂- und/oder -OC(O)CH₂CH₂CH₂-,
A' bedeutet: -CH₂C(O)-, -CH₂CH₂C(O)-, -CH₂CH₂CH₂C(O)-, -C(O)CH₂-, -C(O)CH₂CH₂- und/oder -C(O)CH₂CH₂CH₂- und
E steht für eine Polyalkylenoxidgruppe der allgemeinen Formeln:
   - [CH₂CH₂O]_{q}-[CH₂CH(CH₃)O]ᵣ- und/oder -[OCH(CH₃)CH₂]r,-[OCH₂CH₂]_{q}-, mit q = 1 bis 200 und r = 0 bis 200, wobei das endständige Sauerstoffatom der Gruppe A an die endständige -CH₂-Gruppe der Gruppe E, und das endständige Carbonylkohlenstoffatom der Gruppe A' an das endständige Sauerstoffatom Gruppe E jeweils unter Ausbildung von Estergruppen binden, und/oder mindestens eine endständige Polyalkylenoxid-Struktureinheit der Formel -A-E-R², worin A und E die oben genannte Bedeutung aufweisen, und R² steht für H, geradkettiger, cyclischer oder verzweigter C₁ - C₂₀ - Kohlenwasserstoffrest, der durch -O-, oder-C(O)unterbrochen und mit -OH substituiert und acetylenisch, olefinisch oder aromatisch sein kann,
a2) mindestens einen zweiwertigen oder dreiwertigen organischen Rest, der mindestens eine Ammoniumgruppe enthält,
a3) mindestens eine Polysiloxan- Struktureinheit der allgemeinen Formel:

   -K-S-K-,

   worin S steht für -Si(R¹)₂-O[-Si(R¹)₂-O]ₙ-Si(R¹)₂- und
   worin R¹ steht für C₁-C₂₂-Alkyl, C₁-C₂₂-Fluoralkyl oder Aryl, n steht für 0 bis 1000, und wenn mehrere Gruppen S in der Polysiloxanverbindung vorliegen, diese gleich oder verschieden sein können,
   worin K ein zweiwertiger oder dreiwertiger geradkettiger, cyclischer oder verzweigter C₂-C₄₀-Kohlenwasserstoffrest, der durch -O-, -N -, -NR¹-, -C(O)-, -C(S)-, -N⁺(R³)- und-N⁺(R¹)(R³)- unterbrochen und mit -OH substituiert sein kann,
   worin R¹ wie oben definiert ist, oder gegebenenfalls eine Bindung zu einem zweiwertigen Rest R³ darstellt, und
   worin R³ einen einwertigen oder zweiwertigen geradkettigen, cyclischen oder verzweigten C₁-C₂₀-Kohlenwasserstoffrest, der durch -O-, - NH-, -C(O)-, -C(S)- unterbrochen und mit-OH substituiert sein kann, oder -A-E-R² darstellt, worin A, E und R² wie oben definiert ist, wobei die Reste K gleich oder verschieden voneinander sein können, und im Falle, dass K einen dreiwertigen Rest darstellt, die Absättigung der dritten Valenz über eine Bindung an den vorstehend genannten organischen Rest, der mindestens eine Ammoniumgruppe enthält, erfolgt,
a4) einen organischen oder anorganischen Säurerest zur Neutralisation der aus der(n) Ammoniumgruppe(n) resultierenden Ladungen.

Die erfindungsgemäßen Polysiloxanverbindungen sind dadurch gekennzeichnet, dass sie die vorstehend definierten Komponenten a1) bis a4) aufweisen. Die Polysiloxanverbindungen werden dabei durch Bindung der genannten Struktureinheiten bzw. Reste a1) bis a3) aneinander gebildet. Die Komponente a4) dient der Neutralisation der aus der Komponente a2) resultierenden positiven Ladungen.

Die erfindungsgemäßen Polysiloxanverbindungen können Oligomere oder polymere Verbindungen sein. Oligomere Verbindungen schließen dabei auch den unten beschriebenen Fall ein, worin die Polysiloxanverbindung lediglich eine Wiederholungseinheit aufweist.

Polymere erfindungsgemäße Polysiloxanverbindungen entstehen dabei naturgemäß durch alternierende Verknüpfung von zweiwertigen Resten.

Im Falle der polymeren erfindungsgemäßen Polysiloxanverbindungen resultieren die endständigen Atomgruppierungen aus den endständigen Atomgruppierungen der eingesetzten Ausgangsmaterialien. Dies ist dem Fachmann an sich bekannt.

In einer bevorzugten Ausführungsform sind die polymeren erfindungsgemäßen Polysiloxanverbindungen lineare Polyammonium-Polysiloxanverbindungen, die sich aus den Struktur-Komponenten a1) bis a3) zusammensetzen. So können die linearen polymeren erfindungsgemäßen Polysiloxanverbindungen, insbesondere deren aus den Wiederholungseinheiten gebildete lineare polymere Hauptkette, durch alternierende Aneinanderreihung von Polyalkylenoxid-Struktureinheiten a1), organischen Resten, die mindestens eine, vorzugsweise quartäre Ammoniumgruppe enthalten a2) und Polysiloxan- Struktureinheiten a3) aufgebaut werden. Das heißt, die darüber hinaus gegebenenfalls in den Strukturkomponenten vorhandenen freien Valenzen (wie sie bei dreiwertigen Resten als Komponente a2) oder bei dreiwertigen Resten K auftreten können) dienen bevorzugt nicht dem Aufbau polymerer Seitenketten bzw. polymerer Verzweigungen.

In einer weiteren Ausführungsform kann die Hauptkette der linearen polymeren erfindungsgemäßen Polysiloxanverbindungen von den organischen Resten, die mindestens eine Ammoniumgruppe enthalten a2) und den Polysiloxan - Struktureinheiten a3) aufgebaut werden, und die Polyalkylenoxid- Struktureinheiten a1) binden als Seitenketten an den dreiwertigen organischen Ammoniumgruppenrest.

So können beispielsweise folgende Aufbauten resultieren:
- (Polyalkylenoxidstruktureinheit-Polysiloxanstruktureinheit- Polyalkylenoxidstruktureinheit - bevorzugt quartärer Ammnoniumgruppenrest)ₓ-
- (Polysiloxanstruktureinheit - bevorzugt quartärer Ammoniumgruppenrest)ₓ-Polyalkylenoxidstruktureinheit)ₓ-

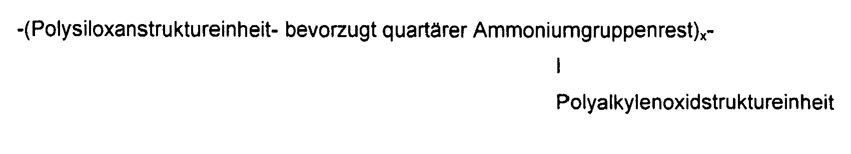

Je nach molarem Verhältnis der monomeren Ausgangsverbindungen können erfindungsgemäße Polysiloxanverbindungen resultieren, die lediglich eine Wiederholungseinheit aufweisen. Dies ist dem Fachmann an sich bekannt. Dieser Fall führt beispielsweise zu erfindungsgemäßen Polysiloxanverbindungen des Aufbaus:
(endständige Polyalkylenoxidstruktureinheit-quartärer Ammoniumgruppenrest-Polysiloxanstruktureinheit-quartärer Ammoniumgruppenrest- endständige Polyalkylenoxidstruktureinheit).

Die erfindungsgemäße Polysiloxanverbindungen bestehen bevorzugt im wesentlichen aus den Komponenten a1) bis a4), wobei die polymeren erfindungsgemäßen Polysiloxanverbindungen naturgemäß die aus der Umsetzung der monomeren Ausgangsmaterialien resultierenden terminalen Gruppen aufweisen. Es können aber auch monofunktionelle Kettenabbruchsmittel eingesetzt werden.

Bei den Polyalkylenoxid-Struktureinheiten a) kann es sich um zweiwertige Reste der allgemeinen Formeln:
- A-E-, -E-A-, -A-E-A'- und/oder -A'-E-A- handeln.

Der Rest A bedeutet dabei:
- CH₂C(O)O-, -CH₂CH₂C(O)O-, -CH₂CH₂CH₂C(O)O-, -OC(O)CH₂-, -OC(O)CH₂CH₂- und/oder -OC(O)CH₂CH₂CH₂-

Der Rest A' bedeutet dabei:
- CH₂C(O)-, -CH₂CH₂C(O)-, - CH₂CH₂CH₂C(O)-, -C(O)CH₂-, -C(O)CH₂CH₂- und/oder -C(O)CH₂CH₂CH₂-.

Die Polyalkylenoxidgruppe E der allgemeinen Formeln:
- [CH₂CH₂O]_{q}-[CH₂CH(CH₃)O]ᵣ- und/oder -[OCH(CH₃)CH₂]ᵣ-[OCH₂CH₂]_{q} mit q = 1 oder 2 bis 200 und r = 0 bis 200, schließen dabei alle möglichen Ethylenoxid/Propylenoxid-Gruppierungen ein. So kann es sich um statistische Ethylenoxid/Propylenoxid-Copolymergruppen oder Ethylenoxid/Propylenoxid-Block Copolymergruppen mit beliebiger Anordnung von einem oder mehreren Ethylenoxid-, oder Propylenoxid-Blöcken handeln.

Die Anbindung der Reste A bzw. A' an die Gruppe E erfolgt dabei so, dass das endständige Sauerstoffatom der Gruppe A an die endständige -CH₂- Gruppe der Gruppe E, und das endständige Carbonyl-Kohlenstoffatom der Gruppe A' an das endständige Sauerstoffatom der Gruppe E jeweils unter Ausbildung von Estergruppen binden.

Bei den Polyalkylenoxid-Struktureinheiten a1) kann es sich weiterhin um einwertige, d.h. endständige Polyalkylenoxid-Struktureinheit der Formel - A-E-R² handeln, worin A und E die oben genannte Bedeutung aufweisen, und R² für H, geradkettiger, cyclischer oder verzweigter C₁-C₂₀-Kohlenwasserstoffrest steht, der durch -O-, oder -C(O)- unterbrochen und mit -OH substituiert und acetylenisch, olefinisch oder aromatisch sein kann.

Die Komponente a2) aus der sich die erfindungsgemäßen Polysiloxanverbindungen zusammensetzen, ist mindestens ein zweiwertiger oder dreiwertiger organischer Rest, der mindestens eine Ammoniumgruppe enthält. Die Bindung des Restes an die übrigen Komponenten der erfindungsgemäßen Polysiloxanverbindungen erfolgt bevorzugt über das Stickstoffatom einer oder mehrerer Ammoniumgruppen in dem organischen Rest. Der Begriff "zweiwertig" bzw. "dreiwertig" bedeutet, dass der organische Ammonium-Rest zur Ausbildung von Bindungen insbesondere zu den übrigen Komponenten der erfindungsgemäßen Polysiloxanverbindungen zwei oder drei freie Valenzen aufweist. Der Ammoniumrest wird zweckmäßig durch eine NH₄⁺-Gruppe dargestellt, in der mindestens zwei Wasserstoffatome durch organische Gruppen substituiert sind. Vorzugsweise handelt es sich um eine sekundäre oder quartäre, besonders bevorzugt um eine quartäre Ammoniumgruppe. Eine quartäre Ammoniumgruppe ist nach allgemeiner Definition (s. z.B. Römpp-Chemie-Lexikon) eine Gruppe bei der alle vier Wasserstoffatome einer NH₄⁺-Gruppe durch organische Reste ersetzt sind.

Die Komponente a2) der erfindungsgemäßen Polysiloxanverbindungen ist mindestens eine Polysiloxan-Struktureinheit der allgemeinen Formel:

-K-S-K-,

S ist darin eine Polysiloxangruppe der allgemeinen Formel

-Si(R¹)₂-O[-Si(R¹)₂-O]ₙ-Si(R¹)₂-,

worin R1 bedeutet C₁-C₂₂-Alkyl, C₁-C₂₂-Fluoralkyl oder Aryl, vorzugsweise Phenyl, n = 0 bis 1000, und wenn mehrere Gruppen S in der Polysiloxanverbindung vorliegen, diese gleich oder verschieden sein können.

R¹ ist bevorzugt C₁-C₁₈-Alkyl, C₁-C₁₈-Fluoralkyl und Aryl. Weiterhin ist R1 bevorzugt C₁-C₁₈-Alkyl, C₁-C₆-Fluoralkyl und Aryl. Weiterhin ist R¹ bevorzugt C₁-C₆-Alkyl, C₁ - C₆-Fluoralkyl, bevorzugter C₁-C₄-Fluoralkyl, und Phenyl. Noch bevorzugter ist R¹ Methyl, Ethyl, Trifluorpropyl und Phenyl.

Der Begriff "C₁-C₂₂-Alkyl" bedeutet im Rahmen der vorliegenden Erfindung, dass die aliphatische Kohlenstoffwasserstoffgruppen 1 bis 22 Kohlenstoffatome besitzen, die geradkettig oder verzweigt sein können. Beispielhaft seien Methyl, Ethyl, Propyl, n-Butyl, Pentyl, Hexyl, Heptyl, Nonyl, Decyl, Undecyl, iso-Propyl, Neopentyl, und 1,2,3 Trimethylhexyl aufgeführt.

Der Begriff "C₁-C₂₂-Fluoralkyl" bedeutet im Rahmen der vorliegenden Erfindung aliphatische Kohlenstoffwasserstoffverbindungen mit 1 bis 22 Kohlenstoffatomen die geradkettig oder verzweigt sein können und mit mindestens einem Fluoratom substituiert sind. Beispielhaft seien Monofluormethyl, Monofluorethyl, 1,1,1-Trifluorethyl, Perflourethyl, 1,1,1-Trifluorpropyl, 1,2,2-Triflourbutyl aufgeführt.

Der Begriff "Aryl "bedeutet im Rahmen der vorliegenden Erfindung unsubstituierte oder ein oder mehrfach mit OH, F, Cl, CF₃ C₁-C₆-Alkyl, C₁-C₆-Alkoxy, C₃-C₇- Cycloalkyl C₂-C₆-Alkenyl oder Phenyl substituiertes Phenyl. Der Ausdruck kann gegebenenfalls auch Naphthyl bedeuten.

K stellt einen zweiwertigen oder dreiwertigen geradkettigen, cyclischen oder verzweigten C₂-C₄₀-Kohlenwasserstoffrest dar, der durch -O-, - NH-, -N-, C(O)-, -C(S)-, -N⁺(R³)-, -NR¹-, und -N⁺(R¹)(R³)- unterbrochen und mit -OH substituiert sein kann.

"Unterbrochen" bedeutet dabei, dass im Falle der zweiwertigen Reste eine - CH₂-Gruppierung im Falle der dreiwertigen Reste eine -CH- Gruppierung des Kohlenwasserstoffrestes durch die genannten Gruppen ersetzt sind. Dies gilt auch für den übrigen Teil der Beschreibung, wenn diese Bezeichnung verwendet wird.

Die Gruppe K bindet über ein Kohlenstoffatom an das Siliziumatom der Gruppe S.

Die Gruppe K kann, wie oben zu sehen, ebenfalls bevorzugt quartäre Ammoniumgruppen aufweisen, so dass Ammoniumgruppen zusätzlich zu den Ammoniumgruppen in der genannten Komponente a2) in den erfindungsgemäßen Polysiloxanverbindungen resultieren.

Die erfindungsgemäßen Polysiloxanverbindungen können, wie zum Beispiel in dem Rest K, Aminogruppen aufweisen. Die Umsetzung der erfindungsgemäßen Polysiloxanverbindungen mit Säuren führt zu deren Protonierung. Solche protonierte Aminogruppen aufweisende Polysiloxanverbindungen sind im Umfang der vorliegenden Erfindung enthalten.

Die Bindung der Komponente a3), der Polysiloxan-Struktureinheit -K-S-K-, zu den übrigen Aufbaukomponenten über den Rest K erfolgt bevorzugt nicht über ein Stickstoffatom des Restes K.

R¹ ist wie oben definiert oder stellt gegebenenfalls eine Bindung zu einem zweiwertigen Rest R³ dar, so dass ein Cyclus resultiert.

R³ stellt einen einwertigen oder zweiwertigen geradkettigen, cyclischen oder verzweigten C₁-C₂₀-Kohlenwasserstoffrest, der durch -O-, -NH-, -C(O)-, -C(S)- unterbrochen und mit -OH substituiert sein kann, oder -A-E-R² dar, worin A, E und R² wie oben definiert ist.

Die Reste K können gleich oder verschieden voneinander sein, und im Falle, dass K einen dreiwertigen Rest darstellt, erfolgt die Absättigung der dritten Valenz über eine Bindung an den - vorstehend genannten organischen Rest, der mindestens eine Ammoniumgruppe enthält.

Die erfindungsgemäßen Polysiloxanverbindungen enthalten weiterhin die Komponente a4), mindestens einen organischen oder anorganischen anionischen Säurerest zur Neutralisation der aus der(n) Ammoniumgruppe(n) resultierenden Ladungen. Organische oder anorganische Säurereste sind Reste, die formal aus der Abspaltung von eines oder mehrerer Protonen aus organischen oder anorganischen Säuren resultieren und schließen beispielsweise ein Halogenide, wie Fluorid, Chlorid, Bromid, Sulfate, Nitrate, Phosphate, Carboxylate, wie Formiat, Acetat, Propionat etc., Sulfonate, Sulfate, Polyethercarboxylate und Polyethersulfate etc. Bevorzugt ist Chlorid. Die organischen oder anorganischen anionischen Säurereste als Komponente a4) der erfindungsgemäßen Polysiloxanverbindungen können gleich oder verschieden voneinander sein. So resultieren aus der Umsetzung der Amine mit Alkylhalogeniden bevorzugt Halogenidionen, während zum Beispiel Carboxylate aus den Carbonsäuren, die bei der. Umsetzung von Bisepoxiden mit Aminen zugesetzt werden können, resultieren.

In einer bevorzugten Ausführungsform der erfindungsgemäßen Polysiloxanverbindungen stellt K einen zweiwertigen oder dreiwertigen geradkettigen, cyclischen oder verzweigten C₂-C₄₀-Kohlenwasserstoffrest dar, der durch -O-, -NH-, -N-, -NR¹-, -C(O)-, -C(S)- unterbrochen und mit-OH substituiert sein kann, worin R¹ wie oben definiert ist, -und wobei die Reste K gleich oder verschieden voneinander sein können.

Der zuvor genannte organische Rest, der mindestens eine, bevorzugt quartäre Ammoniumgruppe enthält, ist bevorzugt ein Rest der allgemeinen Formel:

-N¹-F-N¹-,

worin N¹ eine quartäre Ammoniumgruppe der allgemeinen Formel **-(R** ⁴)N⁺(R⁵)- ist, worin R⁴ einen einwertigen oder zweiwertigen geradkettigen, cyclischen oder verzweigten C₁-C₂₀-Kohlenwasserstoffrest darstellt, der durch -O-, -NH-, -C(O)-, -C(S)- unterbrochen und mit -OH substituiert sein kann, und R⁵ ist ein einwertiger geradkettiger, cyclischer oder verzweigter C₁-C₂₀-Kohlenwasserstoffrest darstellt, der durch -O-, -NH-, -C(O)-, -C(S)- unterbrochen und mit -OH substituiert sein kann, oder eine Einfachbindung zu einem zweiwertigen Rest R⁴ oder einem vierwertigen Rest F, und die Reste R⁴ und R⁵ innerhalb der Gruppe -N¹-F-N¹- sowie in der Polysiloxanverbindung gleich oder verschieden voneinander sein können,

F ist ein zweiwertiger oder vierwertiger geradkettiger, cyclischer oder verzweigter C₂ -C₃₀-Kohlenwasserstoffrest, der durch -O-, -NH-, -N-, - C(O)-, -C(S)-, eine Siloxankette S, wobei für S die oben genannten Bezüge gelten, unterbrochen und mit -OH substituiert sein kann.

Bezüglich weiterer Einzelheiten der Definitionen der quartären Ammoniumgruppe der Formel -N¹-F-N¹- (bevorzugte Ausführungsformen etc.) sei auf die Erläuterungen der ersten Ausführungsform der vorliegenden Erfindung zur Komponente a, den Polyammonium-Polysiloxan Verbindungen, in der diese Gruppe realisiert ist, verwiesen, und die auch in diesem allgemeineren Kontext Gültigkeit besitzen.

Der zuvor genannte organische Rest, der mindestens eine, bevorzugt quartäre Ammoniumgruppe enthält, kann weiterhin bevorzugt ein Rest der allgemeinen Formel
- (R⁶)N⁺(R⁷)- sein,
worin R⁶ ein einwertiger oder zweiwertiger geradkettiger, cyclischer oder verzweigter C₁-C₃₀-Kohlenwasserstoffrest ist, der durch -O-, -NH-, -C(O)-, -C(S)- unterbrochen und mit -OH substituiert sein kann, oder R⁶ stellt eine Einfachbindung zu einem dreiwertigen Rest K dar.

R⁷ ist ein einwertiger geradkettiger, cyclischer oder verzweigter C₁-C₂₀-Kohienwasserstoffrest, der durch -O-, -NH- -C(O)-, -C(S)- unterbrochen und mit -OH substituiert sein kann, oder -A-E-R², worin -A-E-R² die oben genannte Bedeutung aufweist, oder eine Einfachbindung zu einem zweiwertigen Rest R⁶ oder zu einem dreiwertigen Rest K.

Die Reste R⁶ und R⁷ können gleich oder verschieden voneinander sein.

Bezüglich weiterer Einzelheiten der Definitionen der quartären Ammoniumgruppe der Formel-(R⁶)N⁺(R⁷)- (bevorzugte Ausführungsformen) sei auf die Erläuterungen der zweiten, dritten und vierten Ausführungsform zu den erfindungsgemäß bevorzugten Polyammonium-Polysiloxan Verbindungen verwiesen, in der diese Gruppe realisiert ist, und die auch in diesem allgemeineren Kontext Gültigkeit besitzen.

Der zuvor genannte organische Rest, der mindestens eine Ammoniumgruppe enthält, kann weiterhin bevorzugt ein Rest der allgemeinen Formel:
- N⁵-F¹-N⁵- sein,
worin N⁵ eine Ammoniumgruppe der allgemeinen Formel
- (R²³)N⁺(R²⁴)- ist, worin

R²³ Wasserstoff, ein einwertiger oder zweiwertiger geradkettiger, cyclischer oder verzweigter C₁-C₂₀- Kohlenwasserstoffrest darstellt, der durch -O-, -NH-, -C(O)-, -C(S)- unterbrochen und mit -OH substituiert sein kann,
R²⁴ Wasserstoff, ein einwertiger geradkettiger, cyclischer oder verzweigter C₁- C₂₀-Kohlenwasserstoffrest darstellt, der durch -O-, -NH-, -C(O)-, -C(S) unterbrochen und mit -OH substituiert sein kann, oder eine Einfachbindung zu einem zweiwertigen Rest R²³ darstellt, und die Reste R²³ Und R²⁴ innerhalb der Gruppe -N⁵-F¹-N⁵-sowie in der Polysiloxanverbindung gleich oder verschieden voneinander sein können,
F¹ bedeutet ein zweiwertiger geradkettiger, cyclischer oder verzweigter -N Kohlenwasserstoffrest darstellt, der durch -O-, -NH-, -C(O)-, -N-, -C(S)- oder durch eine Gruppe -E- unterbrochen sein kann,
und worin eine Mehrzahl der Gruppen N⁵ und F¹ jeweils gleich oder verschieden voneinander sein können.

Bezüglich weiterer Einzelheiten der Definitionen der Ammoniumgruppe der Formel -N⁵-F¹-N⁵- (bevorzugte Ausführungsformen) sei auf die Erläuterungen der fünften Ausführungsform zur Komponente a, den Polyammonium-Polysiloxan Verbindungen der vorliegenden Erfindung verwiesen, in der diese Gruppe beispielhaft realisiert ist, und die auch in diesem allgemeineren Kontext Gültigkeit besitzen.

Im Folgenden werden die erfindungsgemäß bevorzugten Polyammonium-Polysiloxan-Verbindungen anhand von fünf bevorzugten Ausführungsformen dieser Verbindungen näher beschrieben.

Eine besondere Ausführungsform der Polyammonium-Polysiloxan Verbindungen (die im folgenden als erste Ausführungsform bezeichnet wird), worin der zuvor genannte organische Rest, der mindestens eine, bevorzugt quartäre Ammoniumgruppe enthält, als Komponente a2) der erfindungsgemäßen Polysiloxanverbindungen einen Rest der allgemeinen Formel:

-N¹-F-N¹-

darstellt, wird durch die Polysiloxan-Verbindungen der folgenden allgemeinen Formel (I) dargestellt:

-[B-N¹-F-N¹]m- (I)

worin m = 2 bis 500,
B bedeutet -A-E-K-S-K-E-A- und zusätzlich gegebenenfalls -A-E-A'- bzw. -A'-E-A- ist,
worin S, K, -A-E-, - E-A-, -A-E-A'- bzw. -A'-E-A- und -N¹-F-N¹- wie oben definiert sind, und der Anteil der Gruppe -A-E-A'- bzw. -A'-E-A- in der Gruppe B so gewählt sein kann, dass die Masse von -A-E-A'- bzw. -A'-E-A- von 0 bis 90 %, bevorzugt 0% oder 0,1 bis 50% der Masse des Polysiloxananteils S im Polymer beträgt.

Die erste Ausführungsform der Polyammonium-Polysiloxan Verbindungen betrifft bevorzugt lineare alkylenoxidmodifizierte polyquaternäre Polysiloxane der allgemeinen Formel (I'),

-[B-N¹-F-N¹],- (I')

worin m 2 bis 500,
- B: -A-E-K-S-K-E-A-,
- S: -Si(R¹)₂-O[Si(R¹)₂-O]ₙ-Si(R¹)₂-
- R¹: C₁-C₂₂-Alkyl, C₁-C₂₂-Fluoralkyl oder Aryl,
- n: 0 bis 1000,
- K: ein zweiwertiger geradkettiger, cyclischer oder verzweigter C₂-C₂₀-Kohlenwasserstoffrest, der durch -O-, -NH-, -NR¹-, -C(O)-, -C(S) unterbrochen und mit -OH substituiert sein kann,
- E: eine Polyalkylenoxideinheit der Struktur
- [CH₂CH₂O]_{q} - [CH₂CH(CH₃)O]ᵣ - mit,
- q: 1 bis 200,
- r: 0 bis 200 und
- A: -CH₂C(O)O-, -CH₂CH₂C(O)O- oder - CH₂CH₂CH₂C(O)O-,
- N¹: eine quarternäre Ammoniumstruktur -(R⁴)N⁺(R⁵)-
- R⁴: ein einwertiger oder zweiwertiger geradkettiger, cyclischer oder verzweigter C₁-C₂₀-Kohlenwasserstoffrest darstellt, der durch O-, -NH, -C(O)-, -C(S)- unterbrochen und mit
- -OH: substituiert sein kann,
- R⁵: R⁴ oder eine Einfachbindung zu R⁴ oder F darstellt,
- F: ein zweiwertiger oder vierwertiger geradkettiger, cyclischer oder verzweigter
- C₂-C₃₀-: Kohlenwasserstoffrest darstellt, der durch -O-, -NH-, -N-, -C(O)-, -C(S)- , eine Siloxankette S, wobei für S die oben genannten Bezüge gelten, unterbrochen und mit -OH substituiert sein kann.

Die Möglichkeit einer vierwertigen Substruktur für F bedeutet, dass F ein verzweigtes oder Ringsystem mit den begrenzenden N¹ bilden kann, so dass F dann mit jeweils zwei Bindungen an der Quartärnierung von beiden begrenzenden N¹ beteiligt ist.

Zur näheren Illustration sei auf die Offenlegungsschrift WO 02/10257, insbesondere dort das Beispiel 1, verwiesen.

In einer weiteren Ausführungsform der Polyammonium-Polysiloxan Verbindungen bedeutet die Möglichkeit einer zweiwertigen Substruktur für R⁴, dass es sich in diesen Fällen um eine cyclische Systeme bildende Struktur handelt, worin R⁵ in diesem Fall eine Einfachbindung zu R⁴ ist. Beispiele sind Morpholinyl- und Piperidinylstrukturen.

Bevorzugtere Ausführungsformen dieser so genannten ersten Ausführungsform der Erfindung sowie Verfahren zur Herstellung der genannten Polysiloxanverbindungen der Formel (I) bzw. (I') werden nachfolgend beschrieben.

R⁴ ist bevorzugt -CH₃, -CH₂CH₃, -(CH₂)₂CH₃, -(CH₂)₃CH₃, -(CH₂)₅CH₃, -CH₂CH₂OH,- CH₂CH₂NHCO-R¹⁴ oder -CH₂CH₂CH₂NHCO-R¹⁴, worin
worin R¹⁴ einen geradkettigen, cyclischen oder verzweigten C₁-C₁₈Kohlenwasserstoffrest, der durch -O-, -NH-, -C(O)-, -C(S)- unterbrochen und mit -OH substituiert sein kann, ist.

R⁴ und R⁵ können wie vorstehend erwähnt auch gemeinsam eine cyclische Struktur der Formeln 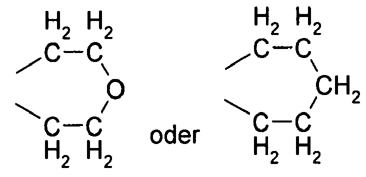 bilden.

Zu den bevorzugten Bedeutungen von R¹ in der so genannten ersten Ausführungsform der Polysiloxanverbindungen kann zu den vorstehenden Ausführungen verwiesen werden.

In der so genannten ersten Ausführungsform der Polysiloxanverbindungen ist R⁴ bevorzugt ein einwertiger oder zweiwertiger geradkettiger, cyclischer oder verzweigter C₁-C₁₆, bevorzugter C₃-C₁₆- Kohlenwasserstoffrest, der durch -O-, -NH-, -C(O)-, -C(S)- unterbrochen und mit -OH substituiert sein kann, bevorzugter ein C₃- C₁₆-Kohtenwasserstoffrest, der durch -O-, -NH-, -NR¹-,-C(O)-, -C(S)- unterbrochen und mit -OH substituiert sein kann, worin R¹ die obengenannte Bedeutung besitzt.

In der so genannten ersten Ausführungsform der Polysiloxanverbindungen ist F bevorzugt ein zweiwertiger oder vierwertiger geradkettiger, cyclischer oder verzweigter C₂-C₂₀-Kohlenwasserstoffrest, der durch -O-, -NH-, -N-, - C(O)-, -C(S), eine Siloxankette S, wobei für S die oben genannten Bezüge gelten, unterbrochen und mit -OH substituiert sein kann.

In der so genannten ersten Ausführungsform der Polysiloxanverbindungen ist K bevorzugt-CH₂CH₂CH₂-, -(CH₂)₄-, -(CH₂)₆-, -CH₂CH₂CH₂OCH₂CH(OH)CH₂-, und -CH=CHCH₂-.

In der so genannten ersten Ausführungsform der Polysiloxanverbindungen stellt R¹⁴ bevorzugt unsubstituierte C₅-C₁₇-Kohlenwasserstoffreste dar, die sich von den entsprechenden Fettsäuren ableiten oder aber hydroxylierte C₃-C₁₇-Reste, die auf hydroxylierte Carbonsäuren, bevorzugt Saccharidcarbonsäuren zurückgeführt werden können.

In der so genannten ersten Ausführungsform sind die erfindungsgemäßen Polyammonium-Polysiloxanverbindungen bevorzugt, bei denen R¹⁴ weiterhin stehen kann für hydroxylierte Reste aus der Gruppe bestehend aus 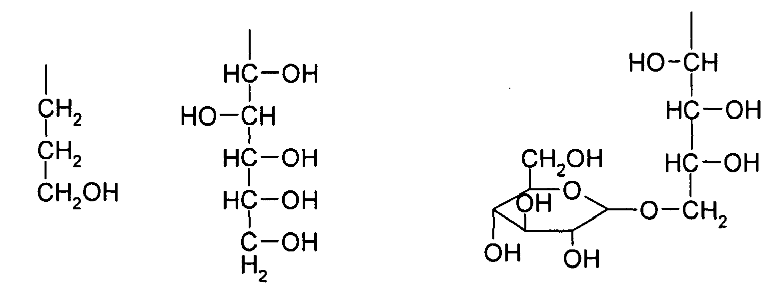 dar.

In der so genannten ersten Ausführungsform der Polysiloxanverbindungen ist m 2 bis 100, bevorzugt 2 bis 50.

In der so genannten ersten Ausführungsform der Polysiloxanverbindungen ist n 0 bis 1000, bevorzugt 0 bis 100, bevorzugter 0 bis 80 und besonders bevorzugt 10 bis 80.

In der so genannten ersten Ausführungsform der Erfindung ist q 1 bis 200, bevorzugt 1 bis 50, bevorzugter 2 bis 20 und besonders bevorzugt 2 bis 10.

In der so genannten ersten Ausführungsform der Erfindung ist r 0 bis 200, bevorzugt 0 bis 100, bevorzugter 0 bis 50 und noch bevorzugter 0 bis 20.

Zur Herstellung der erfindungsgemäßen Polysiloxan-Polyammonium Verbindungen sowohl dieser ersten Ausführungsform als auch aller weiteren bevorzugten Ausführungsformen der erfindungsgemäßen Polysiloxan- Polyammonium Verbindungen sei an dieser Stelle ganz explizit auf die Ausführungsbeispiele der Offenlegungsschrift WO 02/10257 verwiesen.

Eine besondere Ausführungsform der Erfindung (die im Folgenden als so genannte zweite Ausführungsform der Polysiloxanverbindungen bezeichnet wird) wird durch die Polysiloxan Verbindungen der allgemeinen Formel (II) dargestellt,

R²-E-A-N² -K-S-K-N²-A-E-R² (II)

worin
S, K, -A-E-, -E-A- und R² die oben genannten Bedeutungen aufweisen, und N² ein organischer Rest, der mindestens eine quartäre Ammoniumgruppe enthält, der allgemeinen Formel
   - (R⁸)N⁺(R⁹)- ist, worin

   - R⁸: ein einwertiger oder zweiwertiger geradkettiger, cyclischer oder verzweigter C₁-C₂₀-Kohlenwasserstoffrest, der durch -O-, -NH-, -C(O)-, -C(S)- unterbrochen und mit-OH substituiert sein kann,
   - R⁹: ein einwertiger geradkettiger, cyclischer oder verzweigter C₁-C₂₀-Kohlenwasserstoffrest, der durch -O-, -NH-, -C(O)-, -C(S)- unterbrochen und mit-OH substituiert sein kann, oder eine Einfachbindung zu einem zweiwertigen Rest R⁸ oder zu einem dreiwertigen Rest K darstellt, und die Reste R⁸ und R⁹ innerhalb der Polysiloxanverbindung der allgemeinen Formel (II) gleich oder verschieden voneinander sein können.

Bevorzugt handelt es sich bei den Polysiloxanverbindungen der zweiten Ausführungsform um (α,ω-Alkylenoxid- und polyquarternär modifizierte Polysiloxane der allgemeinen Formel (II'),

R¹⁶ - E-A-N²-K-S-K-N²-A-E-R¹⁶ (II') .

Worin die Bezeichnungen stehen für,
- S: -Si(R¹)₂-O[-Si(R¹)₂-O]ₙ-Si(R¹)-
mit R¹ C₁-C₂₂-Alkyl, C₁-C₂₂-Fluoralkyl oder Aryl,
n bedeutet 0 bis 1000,
- K: ein zweiwertiger oder dreiwertiger geradkettiger, cyclischer oder verzweigter C₂-C₂₀-Kohlenwasserstoffrest, der durch -O-, -N-, -NH-, -NR¹-, -C(O)-, -C(S)- unterbrochen und mit -OH substituiert sein kann,
- N²: eine quartäre Ammoniumstruktur -(R⁸)N⁺(R⁹)-
R⁸ ein einwertiger oder zweiwertiger geradkettiger, cyclischer oder verzweigter C₁-C₂₀-Kohlenwasserstoffrest, der durch -O-, -NH-, C(O)-,-C(S)- unterbrochen und mit -OH substituiert sein kann,
R⁹ R⁸ oder eine Einfachbindung zu K oder R⁸,
- A: -CH₂C(O)O-, -CH₂CH₂C(O)O- oder -CH₂CH₂CH₂C(O)O-
- E: eine Polyalkylenoxideinheit der Struktur -[CH₂CH₂O]_{q}-[CH₂CH(CH₃)O]ᵣ-
q 1 bis 200
r 0 bis 200 und
- R¹⁶: H, geradkettiger, cyclischer oder verzweigter C₁-C₂₀-Kohlenwasserstoffrest, der durch -O-, oder -C(O)- unterbrochen und -OH substituiert und acetylenisch, olefinisch oder aromatisch sein kann.

Die Möglichkeit einer dreiwertigen Substruktur für K bedeutet hier, dass K verzweigt sein kann und dann mit zwei Bindungen an der Quartärnierung von N² beteiligt ist. Die Möglichkeit einer zweiwertigen Substruktur für R⁸ bedeutet, dass es sich in diesen Fällen um eine cyclische Systeme bildende Struktur handelt, wobei R⁹ dann eine Einfachbindung zu R² ist.

R⁸ ist bevorzugt -CH₃, -CH₂CH₃, -(CH₂)₂CH₃, -(CH₂)₃CH₃, -(CH₂)₅CH₃, -CH₂CH₂OH -CH₂CH₂NHCO-R¹⁷ oder-CH₂CH₂CH₂NHCO-R¹⁷,
worin R¹⁷ einen geradkettigen, cyclischen oder verzweigten C₁-C₁₈Kohlenwasser-stoffrest, der durch -O-, -NH-, -C(O)-, -C(S)- unterbrochen und mit -OH substituiert sein kann, ist.
R⁸ und R⁹ können wie vorstehend erwähnt auch gemeinsam eine cyclische Struktur der Formeln 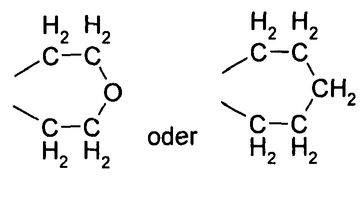 bilden.

Zu den bevorzugten Bedeutungen von R¹ in der so genannten zweiten Ausführungsform der Polysiloxanverbindungen kann zu den vorstehenden Ausführungen verwiesen werden.

In der so genannten zweiten Ausführungsform der Polysiloxanverbindungen ist K bevorzugt ein zweiwertiger oder dreiwertiger geradkettiger, cyclischer oder verzweigter C₃-C₁₆-Kohlenwasserstoffrest, der durch -O-, -NH-, -NR₁-, -N-, -C(O)-, -C(S)- unterbrochen und mit -OH substituiert sein kann, worin R¹ wie vorstehend definiert ist.

Bevorzugt für K sind zum Beispiel Reste der folgenden Strukturen:
- CH₂CH₂CH₂- -CH₂CH₂CH₂OCH₂CHOHCH₂- oder 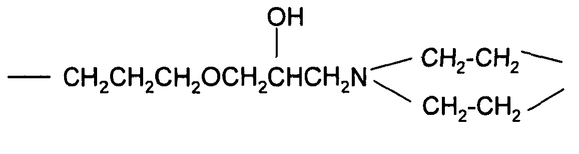
R⁸ ist bevorzugt ein einwertiger oder zweiwertiger geradkettiger, cyclischer oder verzweigter C₁-C₁₆-Kohlenwasserstoffrest, der durch -O-, -NH-, -C(O), -C(S)- unterbrochen und mit -OH substituiert sein kann.

R¹⁶ ist bevorzugt ein geradkettiger, cyclischer oder verzweigter C₁- C₁₈-Kohlen-wasserstoffrest, der durch -O- oder -C(O)- unterbrochen und mit - OH substituiert und acetylenisch oder olefinisch sein kann.

Weiterhin ist R¹⁶ bevorzugt C₅-C₁₇-Alkyl, -CH₂CH=CH₂, -CH₂CH(OH)CH₂OCH₂CH=CH₂, -CH₂CCH, -C(O)CH₃, -C(O)CH₂CH₃.
R¹⁷ stellt bevorzugt unsubstituierte C₅-C₁₇-Kohlenwasserstoffreste, die sich von den entsprechenden Fettsäuren ableiten oder aber hydroxylierte C₃-C₁₇-Reste, die auf hydroxylierte Carbonsäuren, bevorzugt auf Saccharidcarbonsäuren zurückgeführt werden können, dar.

R¹⁷ wird besonders bevorzugt aus der Gruppe aus 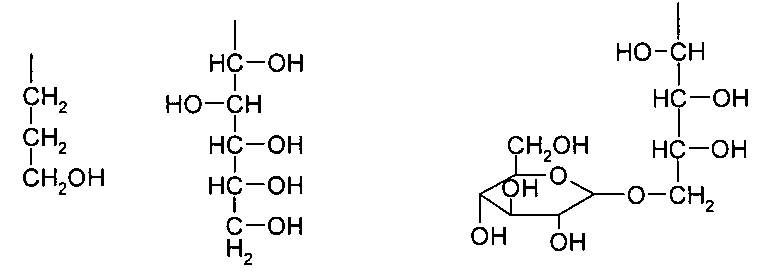 ausgewählt.

In der so genannten zweiten Ausführungsform der Polysiloxanverbindungen ist n bevorzugt 0 bis 200, bevorzugter 0 bis 80, besonders bevorzugt 10 bis 80.

In der so genannten zweiten Ausführungsform der Polysiloxanverbindungen ist q bevorzugt 1 bis 50, bevorzugter 2 bis 20 und besonders bevorzugt 2 bis 10.

In der so genannten zweiten Ausführungsform der Polysiloxanverbindungen ist r bevorzugt 0 bis 100 und bevorzugter 0 bis 50.

In der so genannten zweiten Ausführungsform der Erfindung ist r bevorzugt 0 bis 20 und bevorzugter 0 bis 10.

Zur Herstellung der erfindungsgemäßen Polysiloxan-Verbindungen der so genannten zweiten Ausführungsform sei auf die Ausführungen zur ersten bevorzugten Ausführungsform verwiesen.

Eine besondere Ausführungsform der Polyammonium-Polysiloxan Verbindungen (die im Folgenden als so genannte dritte Ausführungsform der Polysiloxane bezeichnet wird) wird durch die Polysiloxan Verbindungen der allgemeinen Formel (III) dargestellt:

-[K-S-K-N³]m- (III)

in der S, K und m wie oben definiert sind,

N³ ein organischer Rest, der mindestens eine quartäre Ammoniumgruppe enthält, der allgemeinen Formel

-(R¹⁰)-N+(R¹¹)-

ist, worin R¹⁰ ein einwertiger geradkettiger, cyclischer oder verzweigter C₁- C₃₀-Kohlenwasserstoffrest, der durch -O-, -NH-, -C(O)-, -C(S)- unterbrochen und mit -OH substituiert sein kann oder eine Einfachbindung zu K darstellt,
R¹¹ steht für -A-E-R², worin -A-E-R² die oben genannte Bedeutung aufweist.

Bevorzugt handelt es sich bei den Polysiloxanverbindungen der dritten Ausführungsform um Alkylenoxidmodifizierte polyparternäre Polysiloxane der allgemeinen Formel (III'),

-[K-S-K-N³]m- (III'),

in der m 2 bis 500 ist,
- S bedeutet: -Si(R¹)₂-O[-Si(R¹)₂-O]ₙ-Si(R¹)₂-
mit R¹ C₁-C₂₂-Alkyl, C₁-C₂₂-Fluoralkyl oder Aryl,
n = 0 bis 1000,
- N³: eine quartäre Ammoniumstruktur
-(R¹⁰)N⁺(R¹¹)-
worin R¹⁰ ein einwertiger oder zweiwertiger geradkettiger, cyclischer oder verzweigter C₁-C₃₀-Kohlenwasserstoffrest, der durch -O-, -NH-, -C(O)-, -C(S)-unterbrochen und mit -OH substituiert sein kann oder eine Einfachbindung zu K darstellt,
R³ -A-E - ist, mit
A für -CH₂C(O)O-, -CH₂CH₂C(O)O- oder - CH₂CH₂CH₂C(O)O- und
E für eine Polyalkylenoxideinheit der Struktur

-[C H₂CH₂O]_{q}-[C H₂CH(CH₃)O]ᵣ-R¹⁸

q von 1 bis 200,
r von 0 bis 200,
R¹⁸ für H, geradkettiger, cyclischer oder verzweigter C₁-C₂₀-Kohlenwasserstoffrest, der durch -O-, oder -C(O)- unterbrochen -und mit -OH substituiert und acetylenisch, olefinisch oder aromatisch sein kann, sowie
K ist ein zweiwertiger oder dreiwertiger geradkettiger, cyclischer oder verzweigter C₂-C₄₀-Kohlenwasserstoffrest, der durch -O-, -NH-, - NR¹-, -N-, -C(O)-, -C(S)- unterbrochen und mit -OH substituiert sein kann oder eine quartäre Ammonium-struktur N⁵ enthält, mit
N⁵ in der Bedeutung von -(R¹⁹)N⁺(R²⁰)-
R¹⁹ ein einwertiger oder zweiwertiger geradkettiger, cyclischer oder verzweigter C₁-C₂₀-Kohlenwasserstoffrest, der durch -O-, -NH-, -C(O)-, -C(S)- unterbrochen und mit -OH substituiert sein kann oder eine Einfachbindung zu R¹⁰ darstellt, und R²⁰ -A-E- ist, das wie oben definiert ist.

Zur Herstellung der bevorzugten Ausführungsformen der so genannten dritten Ausführungsform der Polysiloxanverbindungen sei wie bereits zuvor explizit auf die Ausführungsbeispiele der Offenlegungsschrift WO 02/10257 verwiesen.

R¹⁰ und R¹⁹ sind unabhängig voneinander bevorzugt -CH₃, -CH₂CH₃, -(CH₂)₂CH₃, -(CH₂)₃CH₃, -(CH₂)₅CH₃, -CH₂CH₂OH, -CH₂CH₂NHCOR²¹ oder -CH₂CH₂CH₂NHCOR²¹, worin R²¹ einen geradkettigen, cyclischen oder verzweigten C₁-C₁₈Kohlenwasserstoffrest, der durch -O-, -NH-,-C(O)-, -C(S)- unterbrochen und mit -OH substituiert sein kann, ist.

In einer Ausführungsform der so genannten dritten Ausführungsform der Polysiloxanverbindungen handelt es sich bei einer zweiwertigen Substruktur für R¹⁰ um eine ein cyclisches System bildende Struktur, wobei R¹⁰ dann eine Einfachbindung zu K besitzt, bevorzugt zu einer tertiären Aminostruktur oder aber zur quartären Struktur N⁵ über R¹⁹.

Zu den bevorzugten Bedeutungen von R¹ in der so genannten dritten Ausführungsform der Polysiloxane kann zu den obigen Ausführungen verwiesen werden.

Bevorzugt ist R¹⁰ ein einwertiger oder zweiwertiger geradkettiger, cyclischer oder verzweigter C₁-C₂₅-Kohlenwasserstoffrest, der durch -O-, -NH-, -C(O)-, -C(S)- unterbrochen und mit -OH substituiert sein kann.

Bevorzugt ist R¹⁹ ein einwertiger oder zweiwertiger geradkettiger, cyclischer oder verzweigter C₁-C₂₅-Kohlenwasserstoffrest, der durch -O-, -NH-, -C(O)-, -C(S)- unterbrochen und mit -OH substituiert sein kann.

In der so genannten dritten Ausführungsform der Polysiloxanverbindungen ist K weiterhin bevorzugt ein zweiwertiger oder dreiwertiger geradkettiger, cyclischer oder verzweigter C₃-C₃₀-Kohlenwasserstoffrest, der durch -O-, -NH-, -NR¹-, -N-, -C(O)-, -C(S)- unterbrochen und mit -OH substituiert sein kann, noch bevorzugter ist K

-CH₂CH₂CH₂OCH₂CHOHCH₂-,

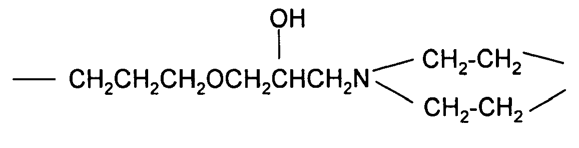

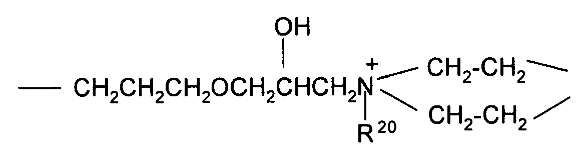

worin R²⁰ wie oben definiert ist.

In der so genannten dritten Ausführungsform der Polysiloxane ist R² bzw. R¹⁸ bevorzugt ein geradkettiger, cyclischer oder verzweigter C₁-C₁₈-Kohlenwasser-stoffrest, der durch -O- oder-C(O)- unterbrochen und -OH substituiert und acetylenisch oder olefinisch sein kann. Bevorzugter ist R² bzw. R¹⁸ C₁-C₆-Alkyl, -CH₂CH=CH₂, -CH₂CH(OH)CH₂OCH₂CH=CH₂, -CH₂CCH, -C(O)CH₃ oder -C(O)CH₂CH₃.

Bevorzugt ist R²¹ ein unsubstituierter C₅-C₁₇-Kohlenwasserstoffrest, der sich von den entsprechenden Fettsäuren ableitet oder aber hydroxylierte C₃-C₁₇-Reste aufweist, und aus der Gruppe von hydroxylierten Carbonsäuren, bevorzugt Saccharidcarbonsäuren stammt.

So ist R²¹ beispielsweise: 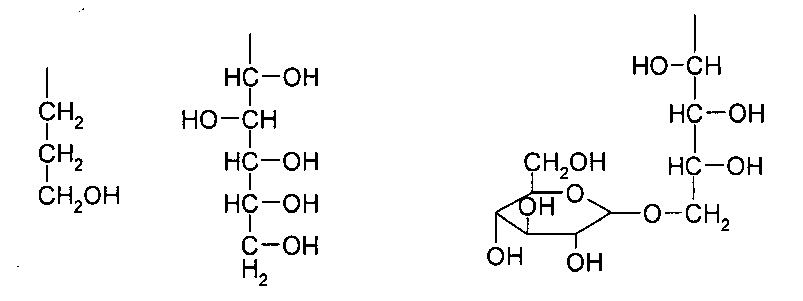 In der so genannten dritten Ausführungsform der Polysiloxane ist m bevorzugt 2 bis 100, und besonders bevorzugt 2 bis 50, n ist 0 bis 100, bevorzugt 0 bis 80, und besonders bevorzugt 10 bis 80, q ist 1 bis 50, bevorzugt 2 bis 50 besonders bevorzugt 2 bis 20, und noch bevorzugter ist q 2 bis 10, r ist 0 bis 100, bevorzugt 0 bis 50, besonders bevorzugt 0 bis 20, und noch bevorzugter ist r 0 bis 10.

Zur Herstellung der erfindungsgemäßen in der Wirkstoffkombination zu verwendenden Polysiloxan-Verbindungen der so genannten dritten Ausführungsform wird zweckmäßig wiederum auf die Ausführungsbeispiele der Offenlegungsschrift WO 02/10257 verwiesen.

Eine besondere Ausführungsform der Polysiloxane (die im Folgenden als so genannte vierte Ausführungsform der erfindungsgemäß zu verwendenden Polysiloxane bezeichnet wird) wird durch die Polysiloxanverbindungen der allgemeinen Formel (IV) dargestellt:

-[N⁴-K-S-K-N⁴-A-E-A']ₘ- bzw. -[N⁴-K-S-K-N⁴-A'-E-A]ₘ- (IV)

worin m, K, S, -A-E-A'- und -A'-E-A- wie oben definiert sind, und
N⁴ ein organischer Rest, der mindestens eine quartäre Ammoniumgruppe enthält, der allgemeinen Formel -(R¹²)N⁺(R¹³)- ist, worin R¹² ein einwertiger oder zweiwertiger geradkettiger, cyclischer oder verzweigter C₁-C₂₀-Kohlenwasserstoffrest ist, der durch -O-, -NH-, -C(O)-, -C(S)-unterbrochen und mit -OH substituiert sein kann,
R¹³ die Bedeutungen von R¹² aufweisen kann, oder eine Einfachbindung zu K oder R¹² darstellt, und die Reste R¹² und R¹³ gleich oder verschieden voneinander sein können.

Bevorzugt handelt es sich bei den Polysiloxanverbindungen der vierten Ausführungsform um Alkylenoxidmodifizierte polyquarternäre Polysiloxane der allgemeinen Formel (IV'),

-[N⁴-K-S-K-N⁴-A-E-A]m- (IV')

worin m = 2 bis 500,
- S: -Si(R¹)₂-O[-Si(R¹)₂-O]-Si(R¹)₂-, worin
R¹ steht für C₁-C₂₂-Alkyl, C₁-C₂₂-Fluoralkyl oder Aryl,
n 0 bis 1000,
- K: einen zweiwertigen oder dreiwertigen geradkettigen, cyclischen oder verzweigten C₂-C₂₀-Kohlenwasserstoffrest darstellt, der durch -O-, -NH-, -NR¹, -N-, -C(O)-, -C(S)- unterbrochen und mit -OH substituiert sein kann,
- N⁴: eine quartäre Ammoniumstruktur -(R¹²)N⁺(R¹³)- ist, worin R¹² ein einwertiger oder zweiwertiger geradkettiger, cyclischer oder verzweigter C₁-C₂₀-Kohlenwasserstoffrest, der durch -O-, -NH-, -C(O)-, -C(S)- unterbrochen und mit -OH substituiert sein kann,
R¹³ steht für R¹² oder eine Einfachbindung zu K oder R¹²,
- A: ist -CH₂C(O)O-, -CH₂CH₂C(O)O- oder -CH₂CH₂CH₂C(O)O-
- E: ist eine Polyalkylenoxideinheit der Struktur -[CH₂CH₂O]_{q}-[CH₂CH(CH₃)O]ᵣ-
mit q = 1 bis 200 und
r = 0 bis 200.
Zu den Herstellungsverfahren sei auf das bisher ausgeführte verwiesen.

Bevorzugtere Ausführungsformen dieser so genannten vierten Ausführungsform Polysiloxane der Formel (IV) bzw. (IV') werden nachfolgend beschrieben.

Die Möglichkeit einer dreiwertigen Substruktur für K bedeutet, dass K verzweigt sein kann und dann mit zwei Bindungen an der Quartarnierung von N⁴ beteiligt sein kann.

Die Möglichkeit einer zweiwertigen Substruktur für R¹² bedeutet, dass es sich in diesen Fällen um eine cyclische Systeme bildende Struktur handelt, wobei R¹³ dann eine Einfachbindung zu R¹² ist.

R¹² ist bevorzugt -CH₃, -CH₂CH₃, -(CH₂)₂CH₃, -(CH₂)₃CH₃, -(CH₂)₅CH₃, -CH₂CH₂OH, -CH₂CH₂NHCOR²² oder -CH₂CH₂CH₂NHCOR²²,
worin R²² einen geradkettigen, cyclischen oder verzweigten C₁-C₁₈Kohlenwasser-stoffrest, der durch -O-, -NH-, -C(O)-, -C(S)- unterbrochen und mit -OH substituiert sein kann, ist.

R¹² und R¹³ können wie vorstehend erwähnt auch gemeinsam eine cyclische Struktur der Formeln 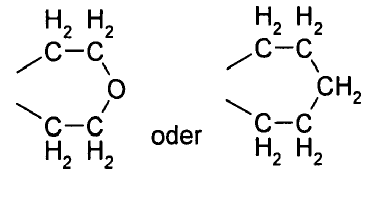 bilden.

Zu den bevorzugten Bedeutungen von R¹ in der so genannten vierten Ausführungsform der Polysiloxane kann auf die vorstehenden Ausführungen verwiesen werden.

Bevorzugt ist R¹² ein einwertiger oder zweiwertiger geradkettiger, cyclischer oder verzweigter C₁-C₁₆-Kohlenwasserstoffrest, der durch -O-, -NH-, -C(O)-, -C(S)- unterbrochen und mit -OH substituiert sein kann.

In der so genannten vierten Ausführungsform, ist K bevorzugt ein zweiwertiger oder dreiwertiger geradkettiger, cyclischer oder verzweigter C₃-C₁₆- Kohlenwasserstoffrest, der durch -O-, -NH-,-NR¹-, -N-, -C(O)-, -C(S)- unterbrochen und mit -OH substituiert sein kann, besonders bevorzugt ist K -CH₂CH₂CH₂-, -CH₂CH₂CH₂OCH₂CHOHCH₂- oder

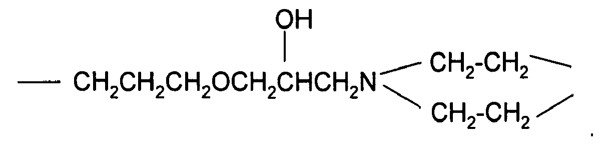

Bevorzugt ist R²² ein unsubstituierter C⁵-C¹⁷-Kohlenwasserstoffrest, der sich von den entsprechenden Fettsäuren ableitet oder aber hydroxylierte C³-C¹⁷-Reste aufweist, die auf hydroxylierte Carbonsäuren, bevorzugt Saccharidcarbonsäuren zurückgeführt worden können.

Bevorzugter ist R²²; 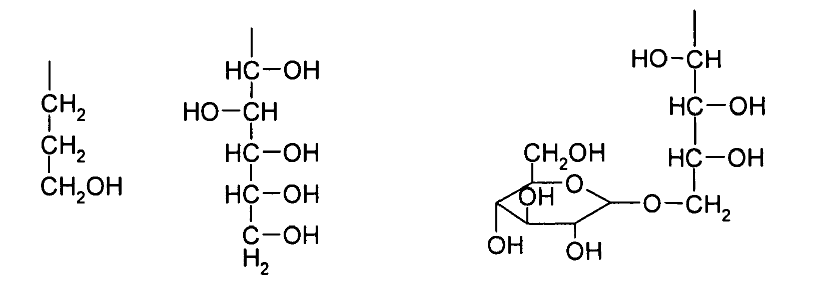 m ist bevorzugt 2 bis 100, und besonders bevorzugt 2 bis 50. n ist 0 bis 100, bevorzugt 0 bis 80, und besonders bevorzugt 10 bis 80. q ist 1 bis 50, bevorzugt 2 bis 50, und besonders bevorzugt 2 bis 20, noch bevorzugter ist q 2 bis 10. r ist 0 bis 100, bevorzugt 0 bis 50, und besonders bevorzugt 0 bis 20, noch bevorzugter ist r 0 bis 10.

Der Begriff "C₁-C₂₂-Alkyl oder C₁-C₃₀-Kohlenwasserstoffrest", wie er vorstehend verwendet wird, bedeutet im Rahmen der vorliegenden Erfindung aliphatische Kohlenstoftwasserstoffverbindungen mit 1 bis 22 Kohlenstoffatomen bzw. 1 bis 30 Kohlenstoffatomen die geradkettig oder verzweigt sein können. Beispielhaft seien Methyl, Ethyl, Propyl, n-Butyl, Pentyl, Hexyl, Heptyl, Nonyl, Decyl, Undecyl, iso Propyl, Neopentyl, und 1,2,3 Trimethylhexyl aufgeführt.

Der Begriff "C₁-C₂₂-Fluoralkyl" bedeutet, wie er vorstehend verwendet wird, im Rahmen der vorliegenden Erfindung aliphatische Kohlenstoffwasserstoffverbindungen mit 1 bis 22 Kohlenstoffatomen die geradkettig oder verzweigt sein können und mit mindestens einem Fluoratom substituiert sind. Beispielhaft seien Monofluormethyl, Monofluorethyl, 1,1,1-Trifluorethyl, Perflourethyl, 1,1,1-Trifluorpropyl, 1,2,2 Triflourbutyl aufgeführt.

Der Begriff "Aryl ", wie er vorstehend verwendet wird, bedeutet im Rahmen der vorliegenden Erfindung unsubstituierte oder ein oder mehrfach mit OH, F, Cl, CF3 C₁-C₆-Alkyl, C₁-C₆-Alkoxy, C₃-C₇- Cycloalkyl, C₂-C₆-Alkenyl oder Phenyl substituiertes Phenyl. Der Ausdruck kann gegebenenfalls auch Naphthyl bedeuten.

Im Rahmen dieser vierten Ausführungsform hat es sich als besonders bevorzugt erwiesen, wenn N⁴ steht für -(R¹²)N⁺(R¹³)-, wobei R¹² steht für eine C₁- bis C₄-Alkylgruppe und R₁₃ steht für eine Einfachbindung zu K.

Weiterhin hat es sich als besonders bevorzugt erwiesen, wenn K steht für einen Rest

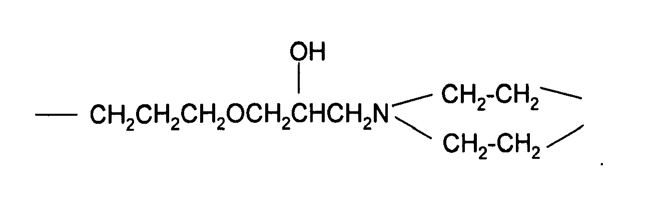

Ebenso kann es erfindungsgemäß bevorzugt sein, wenn S steht für eine Gruppe

-Si(R¹)₂-O[-Si(R¹)₂-O]ₙ-Si(R¹)₂-,

worin R¹ bedeutet C₁-C₂₂-Alkyl, C₁-C₂₂-Fluoralkyl oder Aryl, vorzugsweise Phenyl, n = 0 bis 1000, und wenn mehrere Gruppen S in der Polysiloxanverbindung vorliegen, diese gleich oder verschieden sein können. Weiterhin hat es sich erfindungsgemäß als bevorzugt erwiesen, wenn R¹ jeweils für eine Methylgruppe steht.

Ausserdem kann es erfindungsgemäß bevorzugt sein, wenn A steht für eine Gruppe-CH₂CH₂C(O)O- , A' steht für eine Gruppe -CH₂CH₂C(O)-, und E steht für eine Polyalkylengruppe der allgemeinen Formel -[CH₂CH₂O]_{q}- mit q = 1 bis 200 wobei das endständige Sauerstoffatom der Gruppe A an die endständige -CH₂-Gruppe der Gruppe E, und das endständige Carbonylkohlenstoffatom der Gruppe A' an das endständige Sauerstoffatom Gruppe E jeweils unter Ausbildung von Estergruppen binden.

Eine besondere Ausführungsform der erfindungsgemäßen Polysiloxane (die im Folgenden als so genannte fünfte Ausführungsform der Polysiloxane bezeichnet wird) wird durch die Polysiloxane der allgemeinen Formel (V) dargestellt:

[-N⁵-F¹-N⁵-Y-]ₘ

worin
Y eine Gruppe der Formel -K-S-K- und -A-E-A'- bzw. -A'-E-A- ist,
worin m, K, S, -A-E-A'- und -A'-E-A- wie oben definiert sind, die Gruppen K, S, -A-E-A'- und -A'-E-A- innerhalb der Polysiloxane der allgemeinen Formel (V) gleich oder verschieden voneinander sein können, und das molare Verhältnis der Gruppe -K-S-K- und der Gruppe -A-E-A'- bzw. -A'-E-A- in der Polysiloxanverbindung der allgemeinen Formel (V) von 100: 1 bis 1:100 ist,
- N⁵: eine Ammoniumgruppe der allgemeinen Formel -(R²³)N⁺(R²⁴)- ist,worin
R²³ Wasserstoff, ein einwertiger oder zweiwertiger geradkettiger, cyclischer oder verzweigter C₁-C₂₀-Kohlenwasserstoffrest darstellt, der durch -O-, -NH-, -C(O)-,-C(S)- unterbrochen und mit -OH substituiert sein kann,
- R²⁴: Wasserstoff, ein einwertiger geradkettiger, cyclischer oder verzweigter C₁-C₂₀-Kohlenwasserstoffrest darstellt, der durch -O-, -NH-, - C(O)-, C(S)- unterbrochen und mit -OH substituiert sein kann, oder eine Einfachbindung zu einem zweiwertigen Rest R²³ darstellt, und die Reste R²³ und R²⁴ innerhalb der Gruppe-N⁵-F¹-N⁵- sowie in der Polysiloxanverbindung gleich oder verschieden voneinander sein können,
- F¹: ein zweiwertiger geradkettiger, cyclischer oder verzweigter Kohlenwasserstoffrest darstellt, der durch -O-, -NH-, -N-, -C(O)- oder -C(S)- oder durch eine Gruppe -E- unterbrochen sein kann, worin E wie oben definiert ist, und worin eine Mehrzahl von N⁵ und F¹ jeweils gleich oder verschieden voneinander sein können.
Das molare Verhältnis der Gruppe -K-S-K- und der Gruppe -A-E-A'- bzw. -A'-E-A- in der Polysiloxanverbindung der allgemeinen Formel (V) liegt zwischen 100: 1 und 1: 100. Dieses molare Verhältnis kann wie in der Offenlegungsschrift WO 02/10257 gezeigt, durch die Wahl des molaren Verhältnisses der Ausgangsverbindungen, insbesondere des Verhältnisses der erfindungsgemäß bevorzugt verwendeten (α,ω-Halogencarbonsäurepolyalkylenoxidester-Verbindungen und der Polysiloxan-Bisepoxid-Verbindungen gesteuert werden. Die Eigenschaften der Produkte hängen wesentlich vom verwendeten Verhältnis der Ausgangsmaterialien, sowie der Länge der darin enthaltenen Polyalkylenoxid- bzw. Polysiloxanblöcke ab.

In einer bevorzugten Ausführungsform der so genannten fünften Ausführungsform der Polysiloxane ist K ein zweiwertiger Kohlenwasserstoffrest mit mindestens 4 Kohlenstoffatomen, der eine Hydroxylgruppe aufweist und der durch ein Sauerstoffatom unterbrochen sein kann.

In einer bevorzugten Ausführungsform der so genannten fünften Ausführungsform der Polysiloxane ist F1 ein zweiwertiger geradkettiger, cyclischer oder verzweigter C₂-C₃₀-Kohlenwasserstoffrest, der durch -O-, -NH-, -N-, -C(O)-, -C(S)- oder durch eine Gruppe -E- unterbrochen sein kann, worin E wie oben definiert ist, und worin die Kohlenstoffatome, die aus dem Rest E resultieren, nicht zu den 2 bis 30 Kohlenstoffatomen des C₂-C₃₀ Kohlenwasserstoffrest gezählt werden.

In einer weiteren bevorzugten Ausführungsform der so genannten fünften Ausführungsform der Erfindung ist

-N⁵ -F¹-N⁵-

eine Gruppe der Formel:

-N(R²⁵R²⁶)+-F²-N(R²⁵R²⁶)⁺-

worin
- R²⁵: ein einwertiger oder zweiwertiger geradkettiger, cyclischer oder verzweigter C₁-C₂₀-Kohlenwasserstoffrest, der durch -O-, -NH-, -C(O)-, -C(S)- unterbrochen und mit -OH substituiert sein kann, besonders bevorzugt Methyl ist,
- R²⁶: ein einwertiger geradkettiger, cyclischer oder verzweigter C₁-C₂₀-Kohlenwasserstoffrest, der durch -O-, -NH-, -C(O)-, -C(S) unterbrochen und mit -OH substituiert sein kann, besonders bevorzugt Methyl ist, oder eine Einfachbindung zu einem zweiwertigen Rest R²⁵ darstellt, und die Reste R²⁵ und R²⁶ innerhalb der Gruppe -N⁵-F²-N⁵- sowie in der Polysiloxanverbindung gleich oder verschieden voneinander sein können, und
- F²: ein zweiwertiger geradkettiger, cyclischer oder verzweigter Kohlenwasserstoffrest ist, der durch -O-, -NH-, -N-, -C(O)-, -C(S)- unterbrochen sein kann.
In einer noch bevorzugteren Ausführungsform ist F² eine verzweigte, bevorzugt geradkettige C₁-C₆**-** Alkandiyl-Gruppe, worunter eine 1,6-Hexandiyl- (bzw. Hexamethylen-) Gruppe bevorzugt ist.

In einer weiteren bevorzugten Ausführungsform der so genannten fünften Ausführungsform der Polysiloxanverbindungen ist

-N⁵-F¹-N⁵-

eine Gruppe der Formel:

-N(R²⁷R²⁸)⁺-F³-N(R²⁷R²⁸)⁺-

worin
R²⁷ und R²⁸ jeweils Wasserstoff, C₁-C₆-Alkyl oder Hydroxy(C₁-C₆)alkyl, bevorzugt Wasserstoff, Methyl oder -CH₂CH₂OH sind, und
F³ ein zweiwertiger geradkettiger, cyclischer oder verzweigter Kohlenwasserstoffrest ist, der durch eine Gruppe -E- unterbrochen ist, worin E wie oben definiert ist.

F³ ist besonders bevorzugt eine Gruppe der Formel

-D-E-D-

worin E wie oben definiert ist und D jeweils eine Einfachbindung oder eine geradkettige oder verzweigte C¹-C⁶-Alkandiylgruppe ist, mit der Maßgabe, das D keine Einfachbindung ist, wenn es an ein endständiges Sauerstoffatom der Gruppe E bindet.

Bevorzugt wird die Gruppe -D-E-D- durch eine Gruppe der Formel

-D-(OCH₂CH₂)ᵥ(OCH₂CH(CH₃))_{w}-O-D-

dargestellt, worin D eine geradkettige oder verzweigte C₁ -C₆-Alkandiylgruppe ist und r und q wie oben definiert sind. In der Gruppe -D-(OCH₂CH₂)_{q}(OCH₂CH(CH₃))ᵣ-O-D- können die Ethylenoxid- und Propylenoxideinheiten beliebig angeordnet sein, z.B. als statistische Copolymereinheit oder als Blockcopolymereinheit.

v ist bevorzugt 1 bis 100, bevorzugter 1 bis 70, noch bevorzugter 1 bis 40.

w ist bevorzugt 0 bis 100, bevorzugter 0 bis 70, noch bevorzugter 0 bis 40.

In einer weiteren bevorzugten Ausführungsform der so genannten fünften Ausführungsform der Erfindung wird die Gruppe

-N⁵-F¹-N⁵

durch eine Gruppe der Formel:

-N⁺R²⁵R²⁶-F²-N⁺R²⁵R²⁶-

und eine Gruppe der Formel:

-N⁺R²⁷R²⁸-F³-N⁺R²⁷R²⁸-

dargestellt, worin die Substituenten jeweils die vorstehenden Bedeutungen aufweisen.

Dies bedeutet, das die Polysiloxanverbindungen der allgemeinen Formel (V) aus zwei verschiedenen Typen der Gruppe -N⁵-F¹-N⁵- aufgebaut sind.

In dieser Ausführungsform beträgt das molare Verhältnis der Gruppe

-N⁺R²⁵R²⁶-F²-N⁺R²⁵R²⁶-

zur Gruppe

-N⁺R²⁷R²⁸-F³-N⁺R²⁷R²⁸-

zweckmäßig 70 : 30 bis 95 : 5, bevorzugt 80 : 20 bis 90 : 10.

Die Polysiloxanverbindungen der allgemeinen Formel (V) können cylisch oder linear sein. Im Falle der linearen Verbindungen resultieren die endständigen Gruppen entweder aus den für die Herstellung verwendeten unten beschriebenen bifunktionellen Monomeren oder deren funktionalisierten Derivaten oder aus Monoaminen, die während der Polymerisation als Kettenabbruchmittel zugesetzt werden. Die aus der Verwendung der Monoamin-Kettenabbruchmittel resultierenden terminalen Gruppen liegen bevorzugt als Ammoniumgruppen, entweder durch Quartärnierung oder Protonierung vor.

In einer weiteren bevorzugten Ausführungsform der so genannten fünften Ausführungsform der Polysiloxane steht K für eine der Gruppen der Formel:

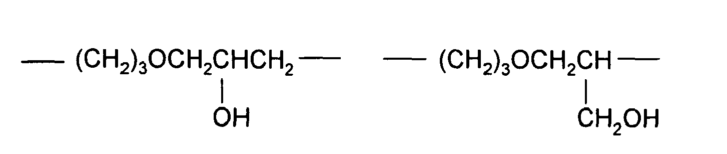

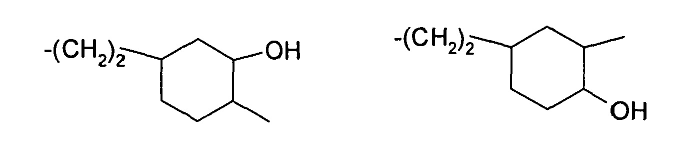

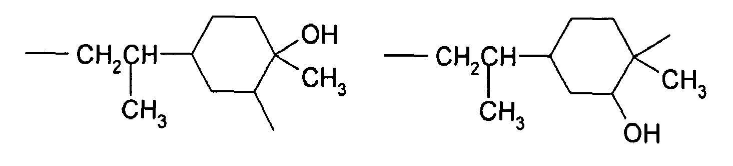

In der so genannten fünften Ausführungsform der Polysiloxane liegt q bevorzugt im Bereich von 1 bis 50, insbesondere 2 bis 50, speziell 2 bis 20 und ganz speziell 2 bis 10, und r liegt im Bereich von 0 bis 100, insbesondere 0 bis 50, speziell 0 bis 20 und ganz speziell 0 bis 10.

In der so genannten fünften Ausführungsform der Erfindung wird der organische oder anorganische Säurerest zur Neutralisation der aus der(n) Ammoniumgruppe(n) resultierenden Ladungen zweckmäßig ausgewählt aus anorganischen Resten, wie Chlorid, Bromid, Hydrogensulfat, Sulfat, bzw. organischen Resten, wie Acetat, Propionat, Octanoat, Decanoat, Dodecanoat, Tetradecanoat, Hexadecanoat, Octadecanoat und Oleat, wobei wie eingangs erwähnt Chlorid und Bromid bevorzugt aus der Umsetzung der Alkylhalogenidgruppen mit Amingruppen resultieren.

Weiterhin liegen die Polysiloxane der fünften Ausführungsform in protonierter Form als Aminsalze oder als Amine vor.

Die Polysiloxane der fünften Ausführungsform der Erfindung werden zweckmäßig hergestellt durch eines der Verfahren, welche in der Offenlegungsschrift WO 02/10257 beschrieben sind.

Die vorstehend beschriebenen Polyammonium-Polysiloxan Verbindungen können beispielsweise unter der Handelsbezeichnung Baysilone^{®} von GE Bayer Silicones bezogen werden. Die Produkte mit den Bezeichnungen Baysilone TP 3911, SME 253 und SFE 839 sind dabei bevorzugt. Ganz besonders bevorzugt ist die Verwendung von Baysilone TP 3911. Weiterhin bevorzugt sind die Polyammonium-Polysiloxan Verbindungen, wie sie in Beispiel 8 der WO 02/10257 beschrieben sind. Dabei sollen erfindungsgemäß auch solche Verbindungen besonders bevorzugt sein, die sich von der in Beispiel 8 der WO 02/10257 genannten Verbindung nur durch eine Variation der Länge der Silikonkette und/oder eine Variation der Länge der Polyetherkette unterscheiden.

Die vorstehend beschriebenen Polyammonium-Polysiloxan Verbindungen werden erfindungsgemäß vorzugsweise in einer Menge von 0,01 bis 10 Gew.% , vorzugsweise 0,01 bis 7,5, besonders bevorzugt 0,01 bis 5,0 Gew.%, ganz besonders bevorzugt von 0,05 bis 2,5 Gew.% jeweils in Bezug auf die Gesamtzusammensetzung verwendet.

### Oxidationsmittel

Weiterhin enthalten die erfindungsgemäßen Mittel zur aufhellenden Farbveränderung keratinischer Fasern mindestens ein Oxidationsmittel, ausgewählt aus Wasserstoffperoxid und dessen Anlagerungsverbindungen an feste Träger.

Als erfindungsgemäßes Oxidationsmittel wird bevorzugt Wasserstoffperoxid selbst verwendet. Das Wasserstoffperoxid wird als Lösung oder in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidon.n H₂O₂ (n ist eine positive ganze Zahl größer 0), Harnstoffperoxid und Melaminperoxid, eingesetzt.

Erfindungsgemäß ganz besonders bevorzugt sind wässrige Wasserstoffperoxid-Lösungen. Die Konzentration einer Wasserstoffperoxid-Lösung wird einerseits von den gesetzlichen. Vorgaben und andererseits von dem gewünschten Effekt bestimmt; vorzugsweise werden 6- bis 12-prozentige Lösungen in Wasser verwendet.

### Bleichkraftverstärker

Weiterhin enthalten die erfindungsgemäßen Mittel zur aufhellenden Farbveränderung keratinischer Fasern mindestens einen Bleichkraftverstärker. Vorzugsweise ist der Bleichkraftverstärker ausgewählt aus Ammoniumpersulfat, Alkalimetallpersulfaten, Ammoniumperoxomonosulfat, Alkalimetallhydrogenperoxomonosulfaten, Alkalimetallperoxidiphosphaten und Erdalkalimetallperoxiden. Besonders bevorzugte Bleichkraftverstärker sind Ammoniumperoxidisulfat, Kaliumperoxidisulfat, Natriumperoxidisulfat, Kaliumhydrogenperoxomonosulfat, Kaliumperoxidiphosphat, Magnesiumperoxid und Bariumperoxid.

Weiterhin hat es sich bei den Arbeiten zur vorliegenden Erfindung als besonders bevorzugt erwiesen, wenn die erfindungsgemäßen Mittel mindestens zwei verschiedene Peroxidisulfate enthalten.

Die erfindungsgemäßen wasserfreien Zusammensetzungen anstelle und/oder zusätzlich zu den festen Peroxoverbindungen einen weiteren Bleichkraftverstärker enthalten.

Als Bleichverstärker können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder lsononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Zur weiteren Steigerung der Aufhellleistung können der erfindungsgemäßen Zusammensetzung zusätzlich als Bleichverstärker mindestens eine gegebenenfalls hydratisierte SiO₂-Verbindung zugesetzt. Obwohl bereits geringe Mengen der gegebenenfalls hydratisierten SiO₂-Verbindungen die Aufhellleistung erhöhen, kann es erfindungsgemäß bevorzugt sein, die gegebenenfalls hydratisierten SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der SiO₂-Verbindungen (ohne deren Wasseranteil) in den Mitteln wieder.

Hinsichtlich der gegebenenfalls hydratisierten SiO₂-Verbindungen unterliegt die vorliegende Erfindung prinzipiell keinen Beschränkungen. Bevorzugt sind Kieselsäuren, deren Oligomeren und Polymeren sowie deren Salze. Bevorzugte Salze sind die Alkalisalze, insbesondere die Kalium und Natriumsalze. Die Natriumsalze sind ganz besonders bevorzugt.

Die gegebenenfalls hydratisierten SiO₂-Verbindungen können in verschiedenen Formen vorliegen. Erfindungsgemäß bevorzugt werden die SiO₂-Verbindungen in Form von Kieselgelen (Silicagel) oder besonders bevorzugt als Wasserglas eingesetzt. Diese SiO₂-Verbindungen können teilweise in wäßriger Lösung vorliegen.

Erfindungsgemäß ganz besonders bevorzugt sind Wassergläser, die aus einem Silikat der Formel (SiO₂)ₙ(Na₂O)ₘ(K₂O)ₚ gebildet werden, wobei n steht für eine positive rationale Zahl und m und p stehen unabhängig voneinander für eine positive rationale Zahl oder für 0, mit den Maßgaben, daß mindestens einer der Parameter m oder p von 0 verschieden ist und das Verhältnis zwischen n und der Summe aus m und p zwischen 1:4 und 4:1 liegt.

Neben den durch die Summenformel beschriebenen Komponenten können die Wassergläser in geringen Mengen noch weitere Zusatzstoffe, wie beispielsweise Phosphate oder Magnesiumsalze, enthalten.

Erfindungsgemäß besonders bevorzugte Wassergläser werden unter anderem von der Firma Henkel unter den Bezeichnungen Ferrosil^{®} 119, Natronwasserglas 40/42, Portil^{®} A, Portil^{®} AW und Portil^{®} W und von der Firma Akzo unter der Bezeichnung Britesil^{®} C20 vertrieben.

Als Bleichverstärker können erfindungsgemäß bevorzugt Carbonatsalze, bzw. Hydrogencarbonatsalze eingesetzt werden. Diese sind bevorzugt ausgewählt aus der Gruppe der Ammonium-, Alkali- (insbesondere Natrium- und Kalium-), sowie Erdalkali- (insbesondere Calcium-), -carbonatsalze bzw. -hydrogencarbonatsalze. Besonders bevorzugte Carbonat-, bzw. Hydrogencarbonatsalze sind Ammoniumhydrogencarbonat, Ammoniumcarbonat, Natriumhydrogencarbonat, Dinatriumcarbonat, Kaliumhydrogencarbonat, Dikaliumcarbonat und Calciumcarbonat. Diese besonders bevorzugten Salze können allein oder in deren Mischungen von mindestens zwei Vertretern als Bleichverstärker verwendet werden.

Als Bleichverstärker vom Typ der Monoalkylcarbonate und deren Derivate werden bevorzugt mindestens ein Kohlensäuremonoester und/oder mindestens ein Kohlensäuremonoamid in dem erfindungsgemäßen Verfahren verwendet. Bevorzugt verwendbare Kohlensäuremonoester sind die Kohlensäuremonoester der Formel (IV), 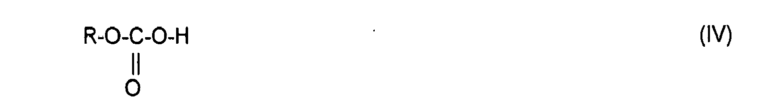 in der R für einen gesättigten oder ungesättigten, geradkettigen, verzweigten, oder cyclischen, substituierten oder unsubstituierten Kohlenwasserstoffrest, oder eine substituierte oder unsubstituierte Arylgruppe bzw. einen substituierten oder unsubstituierten Heterocyclus steht.

In Formel (IV) steht R vorzugsweise für einen substituierten oder unsubstituierten, geradkettigen oder verzweigten Alkyl-, Alkenyl- oder Alkinylrest, wobei als Substituenten bevorzugt Hydroxy-, Amino-, Nitro-, Sulfonsäuregruppen oder Halogene in Frage kommen. Weitere bevorzugte Reste R sind Phenyl- und Benzylreste sowie weiter substituierte Vertreter. Besonders bevorzugt steht R für eine C₁₋₆-Alkylgruppe. Beispiele für erfindungsgemäße C₁-C₆-Alkylgruppen sind die Gruppen Methyl, Ethyl, n-Propyl, Isopropyl, n-Butyl, sec-Butyl, iso-Butyl, tert-Butyl, Pentyl, iso-Pentyl und Hexyl.

Erfindungsgemäß besonders bevorzugt verwendete Zusammensetzungen sind dadurch gekennzeichnet, daß der Rest R in Formel (IV) ausgewählt ist aus Methyl-, Ethyl-, n-Propyl-, iso-Propyl-, n-Butyl-, iso-Butyl-, *tert*-Butyl- sowie Hydroxymethyl- und Hydroxyethyl-Resten.

Alternativ zum Kohlensäuremonoester oder in Verbindung mit ihm können in den wasserfreien Zusammensetzungen Kohlensäuremonoamide als Bleichverstärker eingesetzt werden. Hier ist es erfindungsgemäß bevorzugt, mindestens ein Kohlensäuremonoamid der Formel (V) zu verwenden, 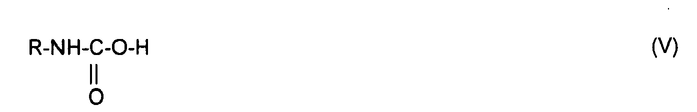 in der R für einen gesättigten oder ungesättigten, geradkettigen, verzweigten, oder cyclischen, substituierten oder unsubstituierten Kohlenwasserstoffrest, oder eine substituierte oder unsubstituierte Arylgruppe bzw. einen substituierten oder unsubstituierten Heterocyclus steht.

In Formel (V) steht R vorzugsweise für einen substituierten oder unsubstituierten, geradkettigen oder verzweigten Alkyl-, Alkenyl- oder Alkinylrest, wobei als Substituenten bevorzugt Hydroxy-, Amino-, Nitro-, Sulfonsäuregruppen oder Halogene in Frage kommen. Weitere Bevorzugte Reste R sind Phenyl- und Benzylreste sowie weiter substituierte Vertreter. Besonders bevorzugt steht R für eine C₁₋₆-Alkylgruppe. Beispiele für erfindungsgemäße C₁-C₆-Alkylgruppen sind die Gruppen Methyl, Ethyl, n-Propyl, Isopropyl, n-Butyl, sec-Butyl, iso-Butyl, tert-Butyl, Pentyl, iso-Pentyl und Hexyl.

Erfindungsgemäß besonders bevorzugte Bleichverstärker der Formel (V) sind dadurch gekennzeichnet, daß der Rest R in Formel (V) ausgewählt ist aus Methyl-, Ethyl-, n-Propyl-, iso-Propyl-, n-Butyl-, iso-Butyl-, *tert*-Butyl- sowie Hydroxymethyl- und Hydroxyethyl-Resten.

Das acide H-Atom des Kohlensäuremonoesters bzw. -monoamids kann auch in neutralisierter Form vorliegen, d.h. es können erfindungsgemäß auch Salze von Kohlensäuremonoestern bzw. Kohlensäuremonoamiden eingesetzt werden. Hier sind erfindungsgemäß Kohlensäuremonoester bzw. das Kohlensäuremonoamide bevorzugt, die in ganz oder teilweise neutralisierter Form, vorzugsweise in Form des Alkalimetall-, Ammonium-, Erdalkalimetall- oder Aluminiumsalzes und insbesondere in Form seines Natriumsalzes, vorliegen.

Als Bleichverstärker vom Typ der Silylcarbonate und deren Derivate werden bevorzugt mindestens ein Silylcarbonat und/oder mindestens ein Silylcarbamat in die erfindungsgemäßen Zusammensetzungen eingearbeitet. Bevorzugt werden Silylcarbonate gemäß Formel (VI) eingesetzt, 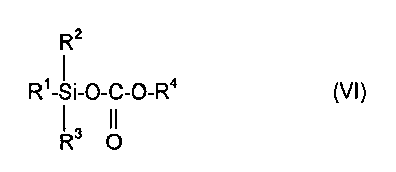 in der die Reste R¹, R² und R³ unabhängig voneinander für ein Wasserstoffatom, einen gesättigten oder ungesättigten, geradkettigen, verzweigten, oder cyclischen, substituierten oder unsubstituierten Kohlenwasserstoffrest oder für eine Trialkylsilylgruppe, vorzugsweise eine Trimethylsilylgruppe oder für eine substituierte oder unsubstiuierte Arylgruppe bzw. einen substituierten oder unsubstituierten Heterocyclus oder für ein Halogen, eine substituierte oder unsubstituierte Hydroxy-, Oxo-, Aminogruppen stehen und der Rest R⁴ für eine chemische Bindung zum Si-Atom oder zu einem der Reste R¹, R² oder R³, ein Wasserstoffatom, einen gesättigten oder ungesättigten, geradkettigen, verzweigten, oder cyclischen, substituierten oder unsubstituierten Kohlenwasserstoffrest oder für eine substituierte oder unsubstituierte Silyl- oder Aluminylgruppe oder für eine substituierte oder unsubstituierte Arylgruppe bzw. einen substituierten oder unsubstituierten Heterocyclus steht.

Bevorzugte Reste R¹, R² und R³ in der oben genannten Formel (VI) sind substituierte oder unsubstituierte, geradkettige oder verzweigte Alkylreste. Unter diesen sind die Alkylreste mit 1 bis 5 Kohlenstoffatomen sowie die Hydroxyalkylreste bevorzugt, so daß bevorzugte erfindungsgemäße wasserfreie Zusammensetzungen dadurch gekennzeichnet sind, daß die Reste R¹, R² und R³ in Formel (VI) ausgewählt sind aus Methyl-, Ethyl-, n-Propyl-, iso-Propyl-, n-Butyl-, iso-Butyl-, *tert-*Butyl- sowie Hydroxymethyl- und Hydroxyethyl-Resten.

Bevorzugte Reste R⁴ in der oben genannten Formel (VI) sind Wasserstoff, substituierte oder unsubstituierte, geradkettige oder verzweigte Alkylreste sowie Trialkylsilylreste. Unter diesen sind Wasserstoff, Methyl-, Ethyl-, tert.-Butyl- und Trimethylsilylreste bevorzugt.

Als Bleichverstärker kann mindestens ein Silylcarbamat der Formel (VII) in der erfindungsgemäßen wasserfreien Zusammensetzung enthalten sein, 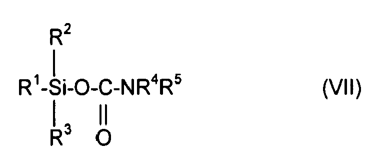 wobei die Reste R¹, R² und R³ unabhängig voneinander für ein Wasserstoffatom, einen gesättigten oder ungesättigten, geradkettigen, verzweigten, oder cyclischen, substituierten oder unsubstituierten Kohlenwasserstoffrest oder für eine Trialkylsilylgruppe, vorzugsweise eine Trimethylsilylgruppe oder für eine substituierte oder unsubstituierte Arylgruppe bzw. einen substituierten oder unsubstituierten Heterocyclus oder für ein Halogen, eine substituierte oder unsubstituierte Hydroxy-, Oxo-, Aminogruppen stehen und die Rest R⁴ und R⁵ unabhängig voneinander für eine chemische Bindung zum Si-Atom oder zu einem der Reste R¹, R² oder R³, ein Wasserstoffatom, einen gesättigten oder ungesättigten, geradkettigen, verzweigten, oder cyclischen, substituierten oder unsubstituierten Kohlenwasserstoffrest oder für eine substituierte oder unsubstituierte Silyl- oder Aluminylgruppe oder für eine substituierte oder unsubstituierte Arylgruppe bzw. einen substituierten oder unsubstituierten Heterocyclus stehen.

Bevorzugte Reste R¹, R² und R³ in der oben genannten Formel (VII) sind substituierte oder unsubstituierte, geradkettige oder verzweigte Alkylreste. Unter diesen sind die Alkylreste mit 1 bis 5 Kohlenstoffatomen sowie die Hydroxyalkylreste bevorzugt, so daß bevorzugt verwendete Zusammensetzungen dadurch gekennzeichnet sind, daß die Reste R¹, R² und R³ in Formel (VII) ausgewählt sind aus Methyl-, Ethyl-, n-Propyl-, iso-Propyl-, n-Butyl-, iso-Butyl-, tert-Butyl- sowie Hydroxymethyl- und Hydroxyethyl-Resten.

Bevorzugte Reste R⁴ und R⁵ in der oben genannten Formel (VII) sind Wasserstoff, substituierte oder unsubstituierte, geradkettige oder verzweigte Alkylreste sowie Trialkylsilylreste. Unter diesen sind Wasserstoff, Methyl-, Ethyl-, tert.-Butyl- und Trimethylsilylreste bevorzugt.

Als weitere zusätzliche Bleichverstärker können in den erfindungsgemäßen Zusammensetzungen bevorzugt mindestens eine Verbindung ausgewählt aus Essigsäure, Milchsäure, Weinsäure, Zitronensäure, Salicylsäure und ortho-Phthalsäure, enthalten sein.

### Weitere Bestandteile

Die Zusammensetzungen enthalten weiterhin bevorzugt mindestens ein Alkalisierungsmittel.
Erfindungsgemäß können die dem Fachmann für Bleich- bzw. Aufhellmittel bekannten, üblichen Alkalisierungsmittel wie Ammonium-, Alkalimetall- und Erdalkalimetallhydroxide, -carbonate,-hydrogencarbonate, -hydroxycarbonate und -carbamide, sowie Alkaliphosphate und Alkalimetasilikate sowie Ammoniak und Alkalihydroxide verwendet werden. Einsetzbar sind erfindungsgemäß aber auch die organischen Amine, wie beispielsweise Monoethanolamin, Arginin, Lysin, Monoethanolamin, Monoisopropanolamin, 2-Amino-2-methylpropanol, 2-Amino-2-methyl-1,3-propandiol, 2-Amino-2-ethyl-1,3-propandiol und 2-Amino-2-methylbutanol.

Die erfindungsgemäßen Zusammensetzungen enthalten die Alkalisierungsmittel bevorzugt in Mengen von 0.2 bis 25 Gew.-%, insbesondere 0.5 bis 10 Gew.-%.

Weiterhin kann es erfindungsgemäß bevorzugt sein, wenn die erfindungsgemäßen Zusammensetzungen mindestens ein Tensid enthalten, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- anionische Alkyl- und/oder Alkenyl-Oligoglykosidcarboxylate, -sulfate, -phosphate und/oder-isethionate, die sich von Alkyl- und/oder Alkenyloligoglykosiden der allgemeinen Formel (IV) ableiten,

   R-O-(G)ₚ (IV)

   mit der Bedeutung
   - R: C₆₋₂₂-Alkyl oder C₆₋₂₂-Alkenyl,
   - G: Glykosideinheit, die sich von einem Zucker mit 5 oder 6 Kohlenstoffatomen ableitet,
   - p: Zahl von 1 bis 10.
   Bevorzugt sind Carboxylate, wie sie beispielsweise unter dem Handelsnamen Plantäpon® LGC (Alkylpolyglucosid-Carboxylat Natriumsalz; 30 % Aktivsubstanz;
   Fa. Cognis) vermarktet werden
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate, anionische Alkyl- und/oder Alkenyl-Oligoglykosidcarboxylate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂-₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolether-gruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.
Beispiele für die in den erfindungsgemäßen Aufhellmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus.
Ebenfalls sehr gut biologisch abbaubar sind quaternäre Esterverbindungen, sogenannte "Esterquats", wie die unter dem Warenzeichen Stepantex^{®} vertriebenen Methylhydroxyalkyldialkoyloxyalkyl-ammoniummethosulfate sowie die unter dem Warenzeichen Dehyquart^{®} vertriebenen Produkte wie Dehyquart^{®} AU-46.

Ein Beispiel für ein als kationisches Tensid einsetzbares quaternäres Zuckerderivat stellt das Handelsprodukt Glucquat^{®}100 dar, gemäß lNCl-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder-alkoholate als Katalysatoren verwendet werden. Die Verwendung von Produkten mit eingeengter Homologenverteilung kann bevorzugt sein.

In einer weiteren Ausführungsform enthalten die erfindungsgemäßen Aufhellmittel zusätzlich zu den Polyammonium-Polysiloxan-Verbindungen weitere strukturverbessernde Wirkstoffe. Diese Wirkstoffe sind bevorzugt in der wasserfreien Zusammensetzung des ersten Erfindungsgegenstandes enthalten, können aber ebenso in der Oxidationsmittelhaltigen Zusammensetzung enthalten sein.

Solche die Haarstruktur verbessernden Wirkstoffe stellen Vitamine und deren Derivate beziehungsweise Vorstufen dar. Erfindungsgemäß besonders bevorzugt sind Panthenol und seine physiologisch verträglichen Derivate. Solche Derivate sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 A1 offenbarten kationischen Panthenolderivate. Ein erfindungsgemäß bevorzugtes Panthenolderivat ist ferner dessen Vorstufe Pantolacton. Panthenol ist innerhalb dieser Gruppe bevorzugt. Ein weiteres Beispiel für ein strukturverbesserndes Vitamin ist Pyridoxin (Vitamin B6).

Weiterhin ist auch Polyvinylpyrrolidon (PVP) für seine faserstrukturverbessernden Eigenschaften bekannt und erfindungsgemäß bevorzugt.

Weitere, erfindungsgemäß besonders wirksame, strukturverbessernde Verbindungen stellen die Aldehyde dar. Besonders bevorzugte Beispiel sind Formaldehyd und Formaldehyd-abspaltende Verbindungen, wie beispielsweise Methoxymethylester, Dimethylol (thio) harnstoffderivate, Oxazolidinderivate, N-Hydroxymethylmaleinimid, Hexamethylentetramin und seine Derivate, Hydantoinderivate, Pyridinium-substituierte Dimethylether, Imidazolidinylharnstoff-Derivate,-Isothiazolinone, 2-Brom-2-nitropropan-diol und 5-Brom-5-nitro-1,3-dioxan. Weitere besonders bevorzugte Aldehyde sind Acetaldehyd, Glyoxal, Glycerinaldehyd und Glutardialdehyd.

Eine weitere geeignete Gruppe von strukturverbessernden Wirkstoffen sind Pflanzenextrakte.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (lKW), Frankfurt, beginnenden Tabelle aufgeführt sind.

Erfindungsgemäß sind vor allem die Extrakte aus Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, grünem Tee, Ginseng und Ingwerwurzel bevorzugt.

Besonders bevorzugt sind die Extrakte aus Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, grünem Tee, Ginseng und Ingwerwurzel.

Ganz besonders für die erfindungsgemäßen Aufhellmittel geeignet sind die Extrakte aus Mandel, Aloe Vera, Kokosnuß, Mango, Aprikose, Limone, Weizen, Kiwi, Melone und grünem Tee.

Wenn die Pflanzenextrakte in die wasserfreie Zusammensetzung des ersten Erfindungsgegenstandes eingearbeitet werden, ist es sinnvoll, die wässrigen Lösemittel zu entfernen.

Weitere strukturverbessernde Wirkstoffe sind Proteinhydrolysate, insbesondere Elastin-, Kollagen-, Keratin-, Milcheiweiß-, Sojaprotein-, Mandelprotein- und Weizen-proteinhydrolysate, deren Kondensationsprodukte mit Fettsäuren sowie quaternisierte Proteinhydrolysate. Besonders bevorzugt sind stark abgebaute Keratinhydrolysate mit Molmassen im Bereich von 400 bis 800. Ferner sind quaternierte Proteinhydrolysate, wie sie beispielsweise unter den Handelsbezeichnungen Gluadin^{®} WQ (INCI-Bezeichnung: Laurdimonium Hydroxypropyl Hydrolyzed Wheat Protein) und Crotein^{®} Q (INCI-Bezeichnung: Hydroxypropyltrimonium Hydrolyzed Collagen) vertrieben werden, erfindungsgemäß besonders bevorzugt. Wenn die Proteinhydrolysate in die wasserfreie Zusammensetzung des ersten Erfindungsgegenstandes eingearbeitet werden, ist es sinnvoll, die wässrigen Lösemittel zu entfernen.

Neben den quaternierten Proteinhydrolysaten stellen auch quaternäre Polymere erfindungsgemäß bevorzugte strukturverbessernde Verbindungen dar. Besonders bevorzugt sind die Polymere, die unter den Handelsbezeichnungen Mirapol^{®} A15 (INCI-Bezeichnung: Polyquaternium-2), Onamer^{®} M (INCI-Bezeichnung: Polyquaternium-1) und Merquat^{®}100 (INCI-Bezeichnung: Polyquaternium-6), Polymer JR 400 (INCI-Bezeichnung: Polyquaternium-10) vertrieben werden.

Ebenfalls faserstrukturverbessernde Wirkstoffe sind Mono-, Di- und Oligosaccharide wie beispielsweise Glucose, Galactose, Fructose, Fruchtzucker, Saccharose und Lactose. Weiterhin können auch Derivate dieser Pentosen, Hexosen und Heptosen, wie die entsprechenden On- und Uronsäuren (Zuckersäuren), Zuckeralkohole, Zuckeramine, wie beispielsweise N-Glucosamin, und Glykoside, sowie mit C₄-C₃₀-Fettalkoholen veretherte Pentosen, Hexosen und Heptosen, erfindungsgemäß eingesetzt werden. Die Zuckersäuren können erfindungsgemäß in freier Form, in Form ihrer Salze, bevorzugt sind Calcium-, Magnesium- und Zink-Salze, und in Form ihrer Ester oder Lactone eingesetzt werden. Bevorzugte Zuckersäuren sind die Gluconsäure, Gluconsäure-y-lacton, Lactobionsäure, die Glucuronsäure und ihre Mono- beziehungsweise Dilactone, die Pangaminsäure, die Zuckersäure, die Mannozuckersäure und ihre Mono- beziehungsweise Dilactone sowie die Schleimsäure und ihre Mono- beziehungsweise Dilactone. Bevorzugte Zuckeralkohole sind Sorbit, Mannit und Dulcit. Bevorzugte Glykoside sind die Methylglucoside. Glucose, N-Glucosamin und Gluconsäure sind aus dieser Gruppe besonders bevorzugt.

Auch gewisse Aminosäuren sind in Rahmen der vorliegenden Erfindung als haarstrukturverbessernde Wirkstoffe einsetzbar. Beispiele sind die in der DE-195 22 569, auf die hier ausdrücklich Bezug genommen wird, beschriebenen Aminosäuren Serin, Threonin und Tyrosin. Ferner sind auch Derivate des Serins, wie beispielsweise das Serinphosphat, erfindungsgemäß bevorzugt. Eine weitere strukturverbessernde Aminosäure stellt Lysin dar. Serin ist ein besonders bevorzugter faserstrukturverbessernder Wirkstoff.

Ebenfalls zur Strukturverbesserung können bestimmte Säuren, insbesondere von Komponente (A2) verschiedene α-Hydroxycarbonsäuren, und ihre Salze eingesetzt werden. Erfindungsgemäß bevorzugte strukturverbessernde Säuren sind Milchsäure, Äpfelsäure, Weinsäure, Glycerinsäure und Maleinsäure. Milchsäure und Glycerinsäure sind besonders bevorzugt. Weiterhin verbessern spezielle Phosphonsäuren und ihre Salze die Struktur keratinhaltiger Fasern. Erfindungsgemäß bevorzugte Phosphonsäuren sind die n-Octylphosphonsäure und die n-Decylphosphonsäure.

Weiterhin kann als strukturverbessernder Wirkstoff Vitamin B₃ eingesetzt werden. Unter dieser Bezeichnung werden häufig die Verbindungen Nicotinsäure und Nicotinsäureamid (Niacinamid) geführt. Erfindungsgemäß bevorzugt ist das Nicotinsäureamid.

Auch Vitamin H ist als strukturverbessernder Wirkstoff im Sinne der vorliegenden Erfindung einsetzbar. Als Vitamin H wird die Verbindung (3a*S*,4*S*, 6a*R*)-2-Oxohexahydrothienol[3,4-*d*]-imidazol-4-valeriansäure bezeichnet, für die sich aber zwischenzeitlich der Trivialname Biotin durchgesetzt hat.

Erfindungsgemäß besonders bevorzugte strukturverbessernde Wirkstoffe sind ausgewählt aus Panthenol, physiologisch verträglichen Panthenol-Derivaten, Mono-, Di- und Oligosacchariden, Serin, Milchsäure, Glycerinsäure, Niacinamid, Vitamin B6, Polyvinylpyrrolidon, Glucose, Gluconsäure und Biotin.

Die erfindungsgemäßen Mittel enthalten die strukturverbessernden Wirkstoffe bevorzugt in Mengen von 0,1 bis 5 Gew.-%, besonders bevorzugt in Mengen von 0,2 bis 2 Gew.-%.

Die erfindungsgemäßen Mittel können weiterhin alle für solche Zubereitungen bekannten Wirk-, Zusatz- und Hilfsstoffe enthalten.

Weiterhin können die erfindungsgemäßen Aufhellmittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.
Bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate.
- polymere Dimethyldiallylammoniumsalze und deren Copolymere mit Acrylsäure sowie Estern und Amiden von Acrylsäure und Methacrylsäure. Die unter den Bezeichnungen Merquat^{®}100 (Poly(dimethyldiallylammoniumchlorid)), Merquat^{®}550 (Dimethyldiallylammoniumchlorid-Acrylamid-Copolymer) und Merquat^{®} 280 (Dimethyldiallylammoniumchlorid-Acrylsäure-Copolymer im Handel erhältlichen Produkte sind Beispiele für solche kationischen Polymere.
- Copolymere des Vinylpyrrolidons mit quaternierten Derivaten des Dialkylaminoacrylats und-methacrylats, wie beispielsweise mit Diethylsulfat quaternierte Vinylpyrrolidon-Dimethylaminomethacrylat-Copolymere. Solche Verbindungen sind unter den Bezeichnungen Gafquat^{®}734 und Gafquat^{®}755 im Handel erhältlich.
- Vinylpyrrolidon-Methoimidazoliniumchlorid-Copolymere, wie sie unter der Bezeichnung Luviquat^{®} angeboten werden.
- quaternierter Polyvinylalkohol
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquaternium-2, Polyquaternium-10 und Polyquaternium-22.

Als konditionierende Wirkstoffe weiterhin geeignet sind Silikonöle, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl, sowie Tocopherolacetat.

Gleichfalls geeignete haarkonditionierende Verbindungen sind Phospholipide, beispielsweise Sojalecithin, Ei-Lecithin und Kephaline.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyltrimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle,
- Cyclodextrine,
- Lösungsmittel und -vermittler wie Ethanol, Isopropanol, Ethylenglykol, Propylenglykol, Glycerin, Dimethylisosorbid und Diethylenglykol,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Im Rahmen einer ersten bevorzugten Ausführungsform wird das erfindungsgemäße Mittel unmittelbar vor der Anwendung durch Mischung einer Zubereitung (A), enthaltend mindestens ein Oxidationsmittel, ausgewählt aus Wasserstoffperoxid und dessen Anlagerungsverbindungen an feste Träger, einer Zubereitung (B) enthaltend mindestens einen Bleichkraftverstärker, und einer Zubereitung (C) hergestellt.

Hinsichtlich der in dieser Ausführungsform bevorzugten Zubereitungen (A) sei an dieser Stelle auf die obigen Ausführungen verwiesen.

Im Rahmen dieser ersten Ausführungsform ist die Zubereitung (B), enthaltend mindestens einen Bleichkraftverstärker, vorzugsweise pulverförmig formuliert.

Im Rahmen dieser ersten Ausführungsform stellt die Zubereitung (C) vorzugsweise eine typische wässrige Cremegrundlage dar.

Ein Beispiel für eine derartige Cremegrundlage stellt die folgende Rahmenrezeptur dar:

| | |
|---|---|
| Fettalkohol / -säure | 5-17% |
| Tensid (z.B. Fettalkoholethersulfat, APG) | 4-15% |
| Emulgator (z.B. Fettalkoholethoxylate) | 0,1-1,2% |
| Alkalisierungsmittel (z. B. Ammoniak / MEA) | 6-10% |
| Komplexbildner und Wasserglas | ca. 1 % |
| Parfüm | 0,2 - 0,8 % |
| Wasser | ad 100 |

Obwohl die erfindungsgemäßen Polyammonium-Polysiloxan-Verbindungen in jede der Zubereitungen A, B oder C eingearbeitet sein können, hat es sich im Rahmen dieser Ausführungsform hat es sich als besonders bevorzugt erwiesen, wenn die erfindungsgemäßen Polyammonium-Polysiloxan-Verbindung in eine der Zubereitungen A oder C eingearbeitet sind. Es ist erfindungsgemäß ganz besonders bevorzugt, wenn die Polyammonium-Polysiloxan-Verbindungen in der Zubereitung C enthalten sind.

Im Rahmen einer zweiten bevorzugten Ausführungsform der vorliegenden Erfindung wird das erfindungsgemäße Mittel unmittelbar vor der Anwendung durch Mischung einer ersten Zubereitung (A), enthaltend mindestens ein Oxidationsmittel, ausgewählt aus Wasserstoffperoxid und dessen Anlagerungsverbindungen an feste Träger, und einer zweiten Zubereitung (B), enthaltend mindestens einen Bleichkraftverstärker, hergestellt.

Hinsichtlich der Zubereitung (A) sei auch an dieser Stelle auf die obigen Ausführungen verwiesen.

Obwohl prinzipiell keine Einschränkungen hinsichtlich der Formulierung der Zubereitung (B) bestehen, hat es sich erfindungsgemäß als bevorzugt erwiesen, wenn die Zubereitung (B) wasserfrei formuliert ist.

Wasserfrei im Sinne der vorliegenden Erfindung bedeutet einen Wassergehalt bezogen auf die Zubereitung (B) von weniger als 5Gew.-%, insbesondere von weniger als 2Gew.-%.
Zubereitungen, die weniger als 0,1Gew.-% Wasser enthalten können erfindungsgemäß ganz besonders bevorzugt sein.

Die Zubereitung (B) ist vorzugsweise als Pulver oder als wasserfreie Paste formuliert.

Im Falle der Formulierung als wasserfreie Paste hat es sich als besonders bevorzugt erwiesen, wenn die Zubereitung (B) mindestens einen nicht hydroxylierten Fettsäureester mit einem Schmelzpunkt von höchstens 50°C, insbesondere von höchstens 30°C, und/oder mindestens eine C₁₀- bis C₃₀-Feftsäure mit mindestens einer zusätzlichen Hydroxygruppe und/oder ein Derivat davon, enthält.

Ester von nicht hydroxylierten C₆- bis C₃₀-Alkylmonocarbonäuren mit C₂- bis C₃₀-Monoalkoholen eignen sich erfindungsgemäß bevorzugt als Fettsäureester. Bevorzugt sind die Monoester der Fettsäuren mit Monoalkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Estern sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit^{®} IPM), lsononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Kokosfettalkohol-caprinat/-caprylat (Cetiol^{®} LC), n-Butylstearat, Oleylerucat (Cetiol^{®} J 600), Isopropylpalmitat (Rilanit^{®} IPP), Oleyloleat (Cetiol^{®}), Laurinsäurehexylester (Cetiol^{®} A), Myristylmyristat (Cetiol^{®} MM), Cetearylisononanoat (Cetiol^{®} SN), Öisäuredecyiester (Cetiol^{®} V).

Die erfindungsgemäßen wasserfreien Zusammensetzungen enthalten die nicht hydroxylierten Fettsäureester (A1) bevorzugt in einer Menge von 2 bis 60 Gew.-%, besonders bevorzugt von 5 bis 30 Gew.-%, jeweils bezogen auf das Gewicht der wasserfreien Zusammensetzung.

Die C₁₀- bis C₃₀-Fettsäure mit mindestens einer zusätzlichen Hydroxygruppe (A2) bzw. deren Derivate können sich von einer gesättigten oder ungesättigten Fettsäure ableiten die bevorzugt ein bis drei zusätzliche Hydroxygruppen tragen. Bevorzugt handelt es sich erfindungsgemäß um gesättigte Fettsäuren bzw. Derivate davon.

Wiederum bevorzugt eignen sich als Fettsäure gemäß (A2) die Monohydroxy-C₁₀- bis C₃₀-Carbonsäuren und deren Derivate. Als Derivate eignen sich insbesondere die Ester mit mindestens einem Vertreter der Fettalkohole, insbesondere derjenigen Fettalkohole, die unter den im Zusammenhang mit den Estern (A1) genannten Fettalkoholanteilen erwähnt werden.
Dabei wird die C₁₀- bis C₃₀-Fettsäure mit mindestens einer zusätzlichen Hydroxygruppe insbesondere ausgewählt aus Verbindungen der Formel (1) und/oder deren physiologisch verträglichen Salzen, 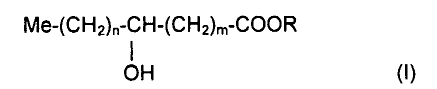 worin
R steht für ein Wasserstoffatom oder eine C₈- bis C₂₂-Alkylgruppe,
m eine ganze Zahl von 0 bis 27 und
n eine ganze Zahl von 0 bis 27 bedeuten, sowie
die Summe m + n eine ganze Zahl von 7 bis 27 beträgt.

Dabei ist es erneut bevorzugt, wenn die C₁₀- bis C₃₀-Fettsäure mit mindestens einer zusätzlichen Hydroxygruppe ausgewählt wird aus solchen Verbindungen der Formel (I) bzw. deren physiologisch verträglichen Salzen, die durch die Parameter
R bedeutet ein Wasserstoffatom oder eine C₈- bis C₂₂-Alkylgruppe,
m eine ganze Zahl von 2 bis 27,
n eine ganze Zahl von 0 bis 27, sowie
die Summe m + n eine ganze Zahl von 7 bis 27
definiert sind.

Solche C₁₀- bis C₃₀-Fettsäuren mit mindestens einer zusätzlichen Hydroxygruppe bzw. deren Derivate sind beispielsweise 12-Hydroxystearinsäure, 3-Hydroxypalmitinsäure, 12-Hydroxylaurinsäure, 16-Hydroxypalmitinsäure und 17-Hydroxypalmitinsäure bzw. deren physiologisch verträglichen Salze.

Ganz besonders bevorzugt ist die C₁₀- bis C₃₀-Fettsäure mit mindestens einer zusätzlichen Hydroxygruppe bzw. deren Derivate 12-Hydroxystearinsäure und/oder eines ihrer Derivate und/oder eines ihrer physiologisch verträglichen Salze. 12-Hydroxystearinsäurederivate sind als Esterderivat insbesondere in hydriertem Rizinusöl enthalten und als solches beispielsweise als Handelsprodukte Cutina^{®} HR (INCI-Bezeichnung: Hydrogenated Castor Oil; Fa. Cognis), Rilanit^{®} HT und Rilanit^{®} GTH (Fa. Cognis) erhältlich.

Die erfindungsgemäßen wasserfreien Zusammensetzungen enthalten die C₁₀- bis C₃₀-Fettsäuren mit mindestens einer zusätzlichen Hydroxygruppe bzw. deren Derivate bevorzugt in einer Menge von 0.1 bis 5 Gew.%, besonders bevorzugt von 0.5 bis 3 Gew.-%, jeweils bezogen auf das Gewicht der wasserfreien Zusammensetzung.

Die erfindungsgemäßen wasserfreien Zusammensetzungen enthalten bevorzugt im Gewichtsverhältnisbereich der nicht hydroxylierten Fettsäureestern (A1) zu den C₁₀- bis C₃₀-Fettsäuren mit mindestens einer zusätzlichen Hydroxygruppe bzw. deren Derivaten (A2) von 10 zu 1 bis 60 zu 1.

Weiter verbessert werden die wasserfreien Zusammensetzungen, durch einen Zusatz mindestens eines Vertreters der Gruppe, bestehend aus Partialglyceriden, Fettalkoholen und Guerbetalkoholen, insbesondere mindestens eines Vertreters der der Gruppe bestehend aus Fettalkoholen und Guerbetalkoholen. Der besagte Zusatz ist bevorzugt in einer Menge von 0.1 bis 20 Gew.-%, besonders bevorzugt von 0.5 bis 10 Gew.-%, jeweils bezogen auf das Gewicht der wasserfreien Zusammensetzung, enthalten.

Partialglyceride, also Monoglyceride, Diglyceride und deren technische Gemische können herstellungs-bedingt noch geringe Mengen Triglyceride -enthalten. Die Partialglyceride folgen vorzugsweise der Formel (II), 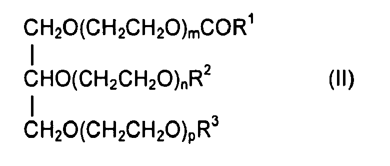 in der COR¹ für einen linearen oder verzweigten, gesättigten und/oder ungesättigten Acylrest mit 6 bis 22, vorzugsweise 12 bis 18 Kohlenstoffatomen, R² und R³ unabhängig voneinander für COR¹ oder OH und die Summe (m+n+p) für 0 oder Zahlen von 1 bis 100, vorzugsweise 5 bis 25 steht, mit der Maßgabe, daß mindestens einer der beiden Reste R² und R³ OH bedeutet.

Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden technische Laurinsäureglyceride, Palmitinsäureglyceride, Stearinsäureglyceride, Isostearinsäureglyceride, Ölsäureglyceride, Behensäureglyceride und/oder Erucasäureglyceride eingesetzt, welche einen Monoglyceridanteil im Bereich von 50 bis 95, vorzugsweise 60 bis 90 Gew.-% aufweisen.

Bevorzugte Fettalkohole sind primäre aliphatische Alkohole der Formel (III),

R¹OH (III)

in der R¹ für einen aliphatischen, linearen oder verzweigten Kohlenwasserstoffrest mit 8 bis 22 Kohlenstoffatomen, der gesättigt ist oder bis zu 3 Doppelbindungen enthalten kann, steht.

Typische Beispiele sind 2-Ethylhexylalkohol, Caprinalkohol, Laurylalkohol, Isotridecylalkohol, Myristylalkohol, Cetylalkohol, Palmoleylalkohol, Stearylalkohol, lsostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen.

Bevorzugt sind Fettalkoholmischungen, die Fettalkohole mit 12 bis 18 Kohlenstoffatomen enthalten, wie beispielsweise solche Mischungen, die aus Kokos-, Palm-, Palmkernöl oder Talgfett, insbesondere aus Kokosöl oder Talgfett, gewonnen werden.

Unter Guerbetalkoholen sind Alkohole zu verstehen, die durch alkalische Kondensation von Alkoholen zu höhermolekularen, verzweigten Iso-Alkoholen hergestellt werden. Diese Umsetzung wurde erstmals von Guerbet 1899 veröffentlicht. Machemer stellte 1952 wesentliche Schritte der Reaktion dar (Angewandte Chemie 64 (1952) 213 - 20): Neben der Dehydrierung zum Keton, bei der Wasserstoff abgespalten wird, und der Aldolkondensation ist die Crotonisierung, bei der Wasser abgespalten wird, ein wichtiger Schritt im Reaktionsablauf. Stand der Technik ist eine Reaktionsführung bei Normaldruck und einer Reaktionstemperatur von 240 bis 260 °C. Die so erhaltenen verzweigten Alkohole werden als Guerbetalkohole bezeichnet. Aus dem Stand der Technik sind inzwischen eine Vielzahl weiterer Verfahren bekannt, gemäß derer man Guerbetalkohole erhalten kann.

Besonders bevorzugt besitzen die Fett- bzw. Guerbetalkohole der erfindungsgemäßen wässrigen Zusammensetzung einen Schmelzpunkt von höchstens 50°C, insbesondere höchstens 30°C.

Ein weiterer Gegenstand der vorliegenden Erfindung ist ein Verfahren zur Blondierung keratinischer Fasern, insbesondere menschlicher Haare, bei dem
- in einem ersten Schritt
   - o: eine Zubereitung A, enthaltend mindestens ein Oxidationsmittel, ausgewählt aus Wasserstoffperoxid und dessen Anlagerungsverbindungen an feste Träger,
   - o: mit einer Zubereitung B, enthaltend mindestens einen Bleichkraftverstärker, und
   - o: gegebenenfalls einer Zubereitung C
   zu einer Anwendungszubereitung vermischt wird,
- diese in einem zweiten Schritt auf die keratinischen Fasern aufgetragen wird und
- anschließend nach einer Einwirkzeit von den Fasern abgespült wird,
wobei mindestens eine der Zubereitungen A, B oder C mindestens eine Polyammonium-Polysiloxan Verbindung enthält.

Im Rahmen dieses Gegenstandes der vorliegenden Erfindung gelten die oben getroffenen Aussagen analog.

So ist es beispielsweise besonders bevorzugt, wenn die Zubereitung A wässrig ist, während die Zubereitung B vorzugsweise wasserfrei formuliert ist. Im Rahmen einer ersten Ausführungsform ist die die Zubereitung B pulverförmig.

### Beispiele

Es wurden die folgenden Zubereitungen hergestellt; sofern keine anderen Angaben gemacht werden, verstehen sich die Werte in Gew.-% bezogen auf die jeweilige Zubereitung.

### 1 Aufhellcreme

| Rohstoff | Menge |
|---|---|
| Eumulgin^{®} B2 | 0,5 |
| Hydrenol^{®} D | 7,5 |
| Lorol^{®} techn. | 3,0 |
| Texapon^{®} NSO | 16,0 |
| Dehyton^{®} K | 10,0 |
| Monoethanolamin | 8,0 |
| L-Arginin | 1,0 |
| Natronwasserglas | 0,5 |
| Turpinal^{®} SL | 0,2 |
| Parfum | 0,6 |
| Silikon | 2,0 |
| Wasser | ad 100 |

### 2 Entwicklerdispersion

| Rohstoff | Menge |
|---|---|
| Lorol^{®} C16 | 3,5 |
| Eumulgin^{®} B2 | 1,0 |
| Disponil^{®} FES 77 | 2,5 |
| Texapon^{®} NSO | 16,0 |
| Dehyton^{®} K | 10,0 |
| Ammoniak 25%ig | 0,7 |
| Dipicolinsäure | 0,1 1 |
| Dinatriumpyrophosphat | 0,03 |
| Turpinal^{®} SL | 1,5 |
| Wasserstoffperoxid 50%ig | 20,0 |
| Aculyn^{®} 33 | 10,0 |
| Wasser | ad 100 |

### 3 Blondaktivator

| Rohstoff | Menge |
|---|---|
| Kaliumpersulfat | 98,6 |
| Aerosil 200 V | 1,4 |

### 4 Versuchsdurchführung

Unmittelbar vor der Anwendung wurden 60 g der Aufhellcreme mit 60 g Entwicklerdispersion und 10 g Blondaktivator miteinander vermischt. Anschließend wurde die resultierende Anwendungszubereitung auf die Strähnen aufgetragen und dort für 30 Minuten bei 32°C belassen. Die Strähnen wurden gründlich mit Wasser gespült und anschließend mit einem Fön getrocknet.

### 5 Verzeichnis der eingesetzten Rohstoffe

- Aculyn^{®} 33: Acrylpolymer (ca. 28% Festkörper in Wasser; INCI-Bezeichnung: Acrylates Copolymer)
- Aerosil^{®} 200: Kieselsäure (INCI-Bezeichnung: Silica) (Degussa)
- Dehyton^{®} K: N,N-Dimethyl-N-(C8-18-kokosamidopropyl)ammoniumaceto-betain (ca. 30% Aktivsubstanz; INCI-Bezeichnung: Aqua (Water), Cocamidopropyl Betaine) (Cognis)
- Disponil^{®} FES 77: Fettalkoholethersulfat (ca. 31-33% Aktivsubstanzgehalt in Wasser; INCl-Bezeichnung: Sodium Coceth-30 Sulfate) (Cognis)
- Eumulgin^{®} B2: Cetylstearylalkohol mit ca. 20 EO-Einheiten (lNCl-Bezeichnung: Ceteareth-20) (Cognis)
- Hydrenol^{®} D: C₁₆₋₁₈-Fettalkohol (lNCl-Bezeichnung: Cetearyl alcohol) (Cognis)
- Lorol^{®} C16: INCI-Bezeichnung: Cetyl Alcohol (Cognis)
- Lorol^{®} tech.: C₁₂₋₁₈-Fettalkohol (lNCl-Bezeichnung: Coconut alcohol) (Cognis)
- Texapon^{®} NSO: Laurylethersulfat, Natriumsalz (ca. 27,5% Aktivsubstanz; INCl-Bezeichnung: Sodium Laureth Sulfate) (Cognis)
- Turpinal^{®} SL: 1-Hydroxyethan-1,1-diphosphonsäure (ca. 58 - 61% Aktivsubstanzgehalt; INCI-Bezeichnung: Etidronic Acid, Aqua (Water)) (Solutia)

## Patentansprüche

1. Mittel zur aufhellenden Farbveränderung keratinischer Fasern, insbesondere menschlicher Haare, enthaltend in einem kosmetisch akzeptablen Träger mindestens ein Oxidationsmittel, ausgewählt aus Wasserstoffperoxid und dessen Anlagerungsverbindungen an feste Träger, und mindestens einen Bleichkraftverstärker, **dadurch gekennzeichnet, dass** es zusätzlich mindestens eine Polyammonium-Polysiloxan Verbindung enthält.

2. Mittel nach Anspruch 1, **dadurch gekennzeichnet, dass** das Polyammonium-Polysiloxan
a1) mindestens eine Polyalkylenoxid-Struktureinheit der allgemeinen Formeln: -A-E-, -E-A-, -A-E-A'- und/oder -A'-E-A- , worin:
A steht für eine der Gruppen: -CH₂C(O)O-, -CH₂CH₂C(O)O-, -CH₂CH₂CH₂C(O)O-, -OC(O)CH₂-, -OC(O)CH₂CH₂- und/oder -OC(O)CH₂CH₂CH₂-,
A' bedeutet: -CH₂C(O)-, -CH₂CH₂C(O)-, -CH₂CH₂CH₂C(O)-, -C(O)CH₂-, -C(O)CH₂CH₂- und/oder -C(O)CH₂CH₂CH₂- und
E steht für eine Polyalkylenoxidgruppe der allgemeinen Formeln:
- [CH₂CH₂O]_{q}-[CH₂CH(CH₃)O]ᵣ- und/oder -[OCH(CH₃)CH₂]ᵣ,-[OCH₂CH₂]_{q}- mit q = 1 bis 200 und r = 0 bis 200, wobei das endständige Sauerstoffatom der Gruppe A an die endständige -CH₂-Gruppe der Gruppe E, und das endständige Carbonylkohlenstoffatom der Gruppe A' an das endständige Sauerstoffatom Gruppe E jeweils unter Ausbildung von Estergruppen binden, und/oder mindestens eine endständige Polyalkylenoxid-Struktureinheit der Formel -A-E-R², worin A und E die oben genannte Bedeutung aufweisen, und R² steht für H, geradkettiger, cyclischer oder verzweigter C₁ - C₂₀ - Kohlenwasserstoffrest, der durch-O-, oder -C(O)unterbrochen und mit -OH substituiert und acetylenisch, olefinisch oder aromatisch sein kann,
a2) mindestens einen zweiwertigen oder dreiwertigen organischen Rest, der mindestens eine Ammoniumgruppe enthält,
a3) mindestens eine Polysiloxan-Struktureinheit der allgemeinen Formel: -K-S-K-,
worin S steht für -Si(R¹)₂-O[-Si(R¹)₂-O]ₙ-Si(R¹)₂- und
worin R¹ steht für C₁-C₂₂-Alkyl, C₁-C₂₂-Fluoralkyl oder Aryl, n steht für 0 bis 1000, und wenn mehrere Gruppen S in der Polysiloxanverbindung vorliegen, diese gleich oder verschieden sein können,
worin K ein zweiwertiger oder dreiwertiger geradkettiger, cyclischer oder verzweigter C₂-C_{4O}-Kohlenwasserstoffrest, der durch -O-, -N -, -NR¹-, -C(O)-, -C(S)-, -N⁺(R³)- und -N⁺(R¹)(R³)- unterbrochen und mit -OH substituiert sein kann,
worin R¹ wie oben definiert ist, oder gegebenenfalls eine Bindung zu einem zweiwertigen Rest R³ darstellt, und
worin R³ einen einwertigen oder zweiwertigen geradkettigen, cyclischen oder verzweigten C₁-C₂₀-Kohlenwasserstoffrest, der durch -O-, - NH-, -C(O)-, -C(S)- unterbrochen und mit -OH substituiert sein kann, oder -A-E-R² darstellt, worin A, E und R² wie oben definiert ist,
wobei die Reste K gleich oder verschieden voneinander sein können, und im Falle, dass K einen dreiwertigen Rest darstellt, die Absättigung der dritten Valenz über eine Bindung an den vorstehend genannten organischen Rest, der mindestens eine Ammoniumgruppe enthält, erfolgt, und
a4) mindestens einen organischen oder anorganischen Säurerest zur Neutralisation der aus der(n) Ammoniumgruppe(n) resultierenden Ladungen
enthält.

3. Mittel nach Anspruch 2, **dadurch gekennzeichnet, dass** das Polyammonium-Polysiloxan eine Verbindung der Formel
-[N⁴-K-S-K-N⁴-A-E-A']ₘ- bzw. -[N⁴-K-S-K-N⁴-A'-E-A]ₘ (IV)
ist, wobei K, S, A, E und A' die oben gegebenen Bedeutungen haben und
N⁴ ein organischer Rest, der mindestens eine quartäre Ammoniumgruppe enthält, der allgemeinen Formel
-(N+R¹²R¹³)-
ist, worin R¹² ein einwertiger oder zweiwertiger geradkettiger, cyclischer oder verzweigter C₁-C₂₀-Kohlenwasserstoffrest ist, der durch -O-, -NH-, -C(O)-, -C(S)- unterbrochen und mit -OH substituiert sein kann,
R¹³ die Bedeutungen von R¹² aufweisen kann, oder eine Einfachbindung zu K oder R¹² darstellt, und die Reste R¹² und R¹³ gleich oder verschieden voneinander sein können.

4. Mittel nach Anspruch 3, **dadurch gekennzeichnet, dass** R¹² steht für eine C₁- bis C₄-Alkylgruppe und R₁₃ steht für eine Einfachbindung zu K.

5. Mittel nach einem der Ansprüche 1 bis 4, **dadurch gekennzeichnet, dass** K steht für einen Rest

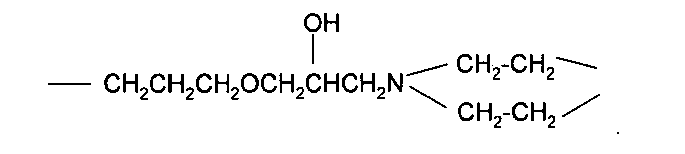

6. Mittel nach einem der Ansprüche 2 bis 5, **dadurch gekennzeichnet, dass** S steht für eine Gruppe
-Si(R¹)₂-O[-Si(R¹)₂-O]ₙ-Si(R¹)₂-,
worin R¹ bedeutet C₁-C₂₂-Alkyl, C₁-C₂₂-Fluoralkyl oder Aryl, vorzugsweise Phenyl, n = 0 bis 1000, und wenn mehrere Gruppen S in der Polysiloxanverbindung vorliegen, diese gleich oder verschieden sein können.

7. Mittel nach Anspruch 6, **dadurch gekennzeichnet, dass** R¹ jeweils für eine Methylgruppe steht.

8. Mittel nach einem der Ansprüche 2 bis 7, **dadurch gekennzeichnet, dass** A steht für eine Gruppe -CH₂CH₂C(O)O- , A' steht für eine Gruppe -CH₂CH₂C(O)-, und E steht für eine Polyalkylengruppe der allgemeinen Formel -[CH₂CH₂O]_{q}- mit q = 1 bis 200 wobei das endständige Sauerstoffatom der Gruppe A an die endständige -CH₂-Gruppe der Gruppe E, und das endständige Carbonylkohlenstoffatom der Gruppe A' an das endständige Sauerstoffatom Gruppe E jeweils unter Ausbildung von Estergruppen binden.

9. Mittel nach einem der Ansprüche 1 bis 8, **dadurch gekennzeichnet, dass** das Oxidationsmittel Wasserstoffperoxid ist.

10. Mittel nach einem der Ansprüche 1 bis 9, **dadurch gekennzeichnet, dass** der Bleichkraftverstärker ausgewählt ist aus Ammoniumpersulfat, Alkalimetallpersulfaten, Ammoniumperoxomonosulfat, Alkalimetallhydrogenperoxomonosulfaten, Alkalimetallperoxidiphosphaten und Erdalkalimetallperoxiden.

11. Mittel nach Anspruch 10, **dadurch gekennzeichnet, dass** der Bleichkraftverstärker ausgewählt ist aus Ammoniumperoxidisulfat, Kaliumperoxidisulfat, Natriumperoxidisulfat, Kaliumhydrogenperoxomonosulfat, Kaliumperoxidiphosphat, Magnesiumperoxid und Bariumperoxid.

12. Mittel nach einem der Ansprüche 1 bis 11, **dadurch gekennzeichnet, dass** als Bleichkraftverstärker mindestens zwei verschiedene Peroxidisulfate enthalten sind.

13. Mittel nach einem der Ansprüche 1 bis 12, **dadurch gekennzeichnet, dass** es zusätzlich mindestens eine gegebenenfalls hydratisierte SiO₂-Verbindung enthält.

14. Mittel nach Anspruch 13, **dadurch gekennzeichnet, dass** die gegebenenfalls hydratisierte SiO₂-Verbindung ein Silikat der Formel (SiO₂)ₙ(Na₂O)ₘ(K₂O)ₚ ist, wobei n steht für eine positive rationale Zahl und m und p stehen unabhängig voneinander für eine positive rationale Zahl oder für 0, mit den Maßgaben, dass mindestens einer der Parameter m oder p von 0 verschieden ist und das Verhältnis zwischen n und der Summe aus m und p zwischen 1:4 und 4:1 liegt.

15. Mittel nach einem der Ansprüche 12 oder 13, **dadurch gekennzeichnet, dass** es die gegebenenfalls hydratisierten SiO₂-Verbindungen in einer Menge von 0.05 bis 15 Gew.% enthält.

16. Mittel nach einem der Ansprüche 1 bis 15 **dadurch gekennzeichnet, dass** zusätzlich mindestens ein Tensid enthalten ist.

17. Verfahren zur Blondierung keratinischer Fasern, insbesondere menschlicher Haare, wobei
- in einem ersten Schritt
o eine Zubereitung A, enthaltend mindestens ein Oxidationsmittel, ausgewählt aus Wasserstoffperoxid und dessen Anlagerungsverbindungen an feste Träger,
o mit einer Zubereitung B, enthaltend mindestens einen Bleichkraftverstärker, und
o gegebenenfalls einer Zubereitung C
zu einer Anwendungszubereitung vermischt wird,
- diese in einem zweiten Schritt auf die keratinischen Fasern aufgetragen wird und
- anschließend nach einer Einwirkzeit von den Fasern abgespült wird,
**dadurch gekennzeichnet, dass** mindestens eine der Zubereitungen A, B oder C mindestens eine Polyammonium-Polysiloxan Verbindung enthält.

18. Verfahren nach Anspruch 17, **dadurch gekennzeichnet, dass** die Zubereitung A wässrig ist.

19. Verfahren nach einem der Ansprüche 17 oder 18, **dadurch gekennzeichnet, dass** die Zubereitung B wasserfrei formuliert ist.

20. Verfahren nach Anspruch 19, **dadurch gekennzeichnet, dass** die Zubereitung B pulverförmig ist.
